# FASCICULE DE BREVET EUROPEEN

(11) **EP 1 194 584 B1**
(45) Date de publication et mention de la délivrance du brevet: **22.02.2006**
(21) Numéro de dépôt: 00949678.7
(22) Date de dépôt: 07.07.2000
(51) Int. Cl.: C12P 19/18, A61K 31/70

(54) **PROCEDE DE PRODUCTION D'OLIGOSACCHARIDES**
VERFAHREN ZUR HERSTELLUNG VON OLIGOSACCHARIDE
METHOD FOR PRODUCING OLIGOPOLYSACCHARIDES

(30) Priorité: 07.07.1999 FR 9908772
(43) Date de publication de la demande: 10.04.2002
(60) Demande divisionnaire: 05111263.9
(73) Titulaire: CENTRE NATIONAL DE LA RECHERCHE SCIENTIFIQUE (CNRS), 75794 Paris Cedex 16 (FR)
(72) Inventeur: SAMAIN, Eric, F-38160 Gières (FR); PRIEM, Bernard, F-38100 Grenoble (FR)
(74) Mandataire: Warcoin, Jacques
(86) Numéro de dépôt international: PCT/FR2000/001972
(87) Numéro de publication internationale: WO 2001/004341

(56) Documents cités:
- EP-A- 0 315 496
- EP-A- 0 392 556
- EP-A- 0 560 409
- WO-A-00/29603
- WO-A-00/70060
- WO-A-95/02683
- WO-A-98/44145
- DE-A- 19 735 994
- BETTLER E ET AL: "The living factory: in vivo production of N-acetyllactosamine containing carbohydrates in E. coli" GLYCOCONJUGATE JOURNAL., vol. 16, mars 1999 (1999-03), pages 205-212, XP002134857 CHAPMAN & HALL., GB ISSN: 0282-0080
- SAMAIN E ET AL: "Production of O-acetylated and sulfated chitooligosaccharides by recombinant Escherichia coli strains harboring different combinations of nod genes" JOURNAL OF BIOTECHNOLOGY,NL,ELSEVIER SCIENCE PUBLISHERS, AMSTERDAM, vol. 72, no. 1-2, 11 juin 1999 (1999-06-11), pages 33-47, XP004172885 ISSN: 0168-1656
- PLUMBRIDGE JACQUELINE ET AL: "Convergent pathways for utilization of the amino sugars N-acetylglucosamine, N-acetylmannosamine, and N-acetylneuraminic acid by Escherichia coli." JOURNAL OF BACTERIOLOGY, vol. 181, no. 1, janvier 1999 (1999-01), pages 47-54, XP000917021 ISSN: 0021-9193 cité dans la demande

## Description

La présente invention a pour objet la production par voire microbiologique d'oligosaccharides d'intérêt biologique.

Il est maintenant bien établi que les oligosaccharides jouent un rôle biologique important notamment au niveau de l'activité et de la fonction des protéines ; ils servent ainsi à moduler la durée de la demi-vie des protéines, parfois ils interviennent dans la structure de la protéine. Les oligosaccharides jouent un rôle critique dans la variabilité antigénique (groupe sanguin par exemple), et dans certaines infections bactériennes telles celles provoquées par *Neisseria meningitidis.*

Comme les oligosaccharides sont habituellement obtenus avec un faible rendement par purification à partir de sources naturelles, la synthèse d'oligosaccharides est devenue un challenge majeur de la chimie des carbohydrates, afin de fournir des quantités suffisantes d'oligosaccharides bien caractérisés, nécessaires à la recherche fondamentale ou pour toutes autres applications potentielles (Boons et *al.,* 1996).

La synthèse d'oligosaccharides complexes d'intérêt biologique peut être réalisée par voie chimique, enzymatique ou microbiologique.

Malgré le développement de nouvelles méthodes chimiques de synthèse d'oligosaccharides au cours de ces 20 dernières années, la synthèse chimique d'oligosaccharides reste très difficile en raison des nombreuses étapes de protections et de déprotections sélectives, de la labilité des liaisons glycosidiques, des difficultés à obtenir des couplages régiospécifiques et des faibles rendements de production. Comme le nombre des étapes augmente avec la taille de l'oligosaccharide, la préparation de larges quantités d'oligosaccharides plus longs que les trisaccharides n'est pas aisée. Contrairement à l'expérience de la synthèse peptidique ou de la synthèse nucléique, la chimie organique synthétique traditionnelle ne peut donc fournir aujourd'hui une synthèse de qualité et en quantité d'oligosaccharides même de formule simple.

Conséquemment, les méthodes enzymatiques sont devenues plus populaires car elles permettent une synthèse régiosélective dans des conditions douces et sans étape de protection des groupes hydroxyles. Le développement de l'approche enzymatique a été rendu possible par le clonage et l'identification fonctionnelle de nombreux gènes codant pour les enzymes intervenant dans la voie de synthèse des oligosaccharides. Ainsi, différents types d'enzymes peuvent être utilisés pour la synthèse *in vitro* d'oligosaccharides. La fonction physiologique des glycosyl-hydrosylases et des glycosyl-phosphorylases est de dépolymériser les oligosaccharides mais elles peuvent également être utilisées *in vitro* dans la synthèse des oligosaccharides en contrôlant l'équilibre et la cinétique de la réaction. Les substrats des enzymes de ces réactions sont aisément disponibles mais ces réactions enzymatiques ne sont pas très versatiles. Une autre méthode enzymatique développée utilise les glycosyl-transférases de la voie biochimique Leloir qui présentent une forte régiospécificité pour le précurseur ainsi que pour le substrat donneur ; ces glycosyl-transférases ne sont pas aussi aisément disponibles que les glycosyl-hydrolases. La technique de l'ADN recombinant, a récemment permis de cloner et de produire un certain nombre d'entre elles. Cependant, la principale limitation de cette méthode enzymatique réside dans le coût très élevé des nucléotides-sucres qui sont les donneurs de sucre utilisé par ces enzymes.

La voie microbiologique de production d'oligosaccharides recombinants *in vivo* est la plus séduisante des voies de synthèse puisque la bactérie se charge à la fois de la biosynthèse des enzymes, de la régénération des nucléotides-sucres et finalement de la production de l'oligosaccharide.

Les premières descriptions de la synthèse d'oligosaccharides par la voie microbiologique utilisant des bactéries recombinantes peuvent être considérées dans une certaine mesure comme les travaux qui ont conduit à l'élucidation des voies de biosynthèse des facteurs de nodulation ; ces facteurs sont des molécules signal sécrétées par les rhizobia pour permettre la reconnaissance par les légumineuses dans le processus de nodulation. Les facteurs de nodulation sont constitués d'un squelette chitooligosaccharidique portant différentes substitutions. L'identification fonctionnelle des gènes nod impliqués dans la biosynthèse des facteurs de nodulation a été en partie réalisée en identifiant les oligosaccharides formés *in vivo* dans des souches d*'Escherichia coli* exprimant ces différents gènes nod (Gérémia *et al,* 1994 ; Kamst *et al.,* 1995 ; Spaink *et al,* 1994 ; Mergaert *et al,* 1995). DE 197 35 994 décrit des microorganismes exprimant une glycosyltransférase, Bettler et al. (1999-03) Glycoconjugate Journal vol. 16 205-212 décrit qu'une faible partie des oligosaccharides produits chez E. coli sont substitués par du glycérol, Samain et al (1999-06) Journal of Biotechnology Vol.72 33-47 décrit la production de chitooligosaccharides acétylés chez E. Coli et WO 98/44145 décrit des souches de E. coli pour la production d'oligosaccharides. Cependant, la production d'oligosaccharides en elle-même n'était pas le but de ces études ; ces produits n'ont été synthétisés qu'à l'état de trace et ne furent identifiés que grâce à l'utilisation de précurseurs radioactifs.

En revanche, il a été récemment démontré dans notre laboratoire (Samain *et al,* 1997) que la culture à haute densité cellulaire de souches *d'Escherichia coli* possédant le gène *nodC* (chitooligosaccharide synthase) permettait de produire des quantités importantes supérieures à 2 g/l de chitooligosaccharides dits recombinants.

Cette technique de synthèse microbiologique d'oligosaccharides reste cependant limitée à la production des seuls chitooligosaccharides, du fait de la propriété unique de nodC (chitooligosaccharide synthase) de fonctionner sans précurseur, les autres enzymes glycosylent en effet un précurseur spécifique et leur activité est donc dépendante de la présence de ce précurseur dans la cellule. Le problème du précurseur est donc le principal verrou qui bloque le développement de la méthode et de son extension à la production d'autres types d'oligosaccharides.

La présente invention a donc pour objet un procédé de production d'un oligosaccharide d'intérêt par une cellule génétiquement modifiée à partir d'au moins un précurseur exogène internalisé par ladite cellule, selectionné parmi le lactose, un β-galactoside et l'acide sialique, ledit précurseur intervenant dans la voie de biosynthèse dudit oligosaccharide, ledit procédé comprenant les étapes (i) d'obtention d'une cellule qui comprend au moins un gène recombinant codant pour un enzyme capable d'effectuer une modification dudit précurseur exogène ou de l'un des intermédiaires de la voie de biosynthèse dudit oligosaccharide à partir dudit précurseur exogène nécessaire à la synthèse dudit oligosaccharide à partir dudit précurseur, ainsi que les éléments permettant l'expression dudit gène dans ladite cellule, ladite cellule étant dépourvue d'activité enzymatique susceptible de dégrader ledit oligosaccharide, ledit précurseur et lesdits intermédiaires ; (ii) de mise en culture de ladite cellule en présence d'au moins undit précurseur exogène, dans des conditions permettant l'internalisation selon un mécanisme de transport passif et/ou actif dudit précurseur exogène par ladite cellule et la production dudit oligosaccharide par ladite cellule.

La présente invention concerne un procédé tel que décrit ci-dessus caractérisé en ce que ladite cellule comprend en outre au moins un gène codant pour un enzyme capable d'effectuer une modification d'un précurseur endogène intervenant dans la voie de biosynthèse dudit oligosaccharide, ledit enzyme étant identique ou différent de l'enzyme utilisé dans le procédé décrit ci-dessus, ainsi que les éléments permettant l'expression dudit gène dans ladite cellule et caractérisé en ce que ladite cellule est dépourvue d'activité enzymatique susceptible de dégrader ledit précurseur.

On entend désigner par oligosaccharides, des polymères linéaires ou ramifiés au nombre variable de résidus, de liaisons et de sous-unités ; le nombre de résidus étant supérieur à 1. Les oligosaccharides sont des glucides qui se transforment à l'hydrolyse en plusieurs molécules de monosaccharides ; les monosaccharides étant les sucres qu'on ne peut transformer par hydrolyse en substance plus simple. On subdivise les monosaccharides en trioses, tétroses, pentoses, hexoses, heptoses selon le nombre d'atomes de carbone de leur chaîne hydrocarbonée et aussi en aldoses et cétoses selon la présence d'une fonction aldéhydique ou d'une fonction cétone dans leur molécule. Parmi les monosaccharides les plus fréquents, on peut citer le mannose, le glucose, le galactose, le N-acétyl-glucosamine, le N-acétyl-galactosamine. Le nombre de chaînes d'oligosaccharides stéréoisomères est extrêmement large, du au nombre important de carbones asymétriques dans la chaîne hydro-carbonée.

Par précurseur exogène, on entend désigner un composé intervenant dans la voie de biosynthèse de l'oligosaccharide selon l'invention qui est internalisé par ladite cellule. Par précurseur endogène, on entend désigner un composé intervenant dans la voie de biosynthèse de l'oligosaccharide selon l'invention qui est naturellement présent dans ladite cellule.

Par cellule génétiquement modifiée, on entend désigner un microorganisme dans lequel au moins une altération de la séquence d'ADN a été introduite dans son génome afin de conférer un phénotype particulier à ladite cellule. De telles altérations peuvent ainsi conférer par exemple l'aptitude de la cellule à ne pas dégrader ou à ne pas modifier un composé selon l'invention ou à ne pas diminuer la fréquence de réarrangement de l'ADN.

Le procédé selon l'invention est caractérisé en ce que ladite cellule est une cellule choisie parmi les bactéries et les levures. Selon un mode de réalisation préférée de l'invention, la bactérie est choisie parmi le groupe composé de *Escherichia coli, Bacillus subtilis, Campylobacter pylori, Helicobacter pylori, Agrobacterium tuméfaciens, Staphylococcus aureus, Thermophilus aquaticus, Azorhizobium caulinodans, Rhizobium leguminosarum, Neisseria gonorrhoeae, Neisseria meningitis.* Selon un mode préféré de réalisation de l'invention, la bactérie est *Escherichia coli.* Selon un autre mode de réalisation de l'invention, la cellule est une levure qui est de préférence *Saccharomyces cerevisiae, Saccharomyces pombe, Candida albicans.* La cellule selon l'invention est dépourvue d'activité enzymatique susceptible de dégrader ledit oligosaccharide, ledit précurseur ou lesdits intermédiaires métaboliques.

La séquence d'acide nucléique codant pour l'enzyme selon l'invention est soit naturellement présente dans ladite cellule soit est introduite dans ladite cellule par les techniques de l'ADN recombinant connues de l'homme du métier. Dans la présente description, on entendra désigner par acide nucléique, un fragment d'ADN, aussi bien double brin que simple brin, que des produits de transcription desdits ADNs, et/ou un fragment d'ARN. Selon un mode préféré de réalisation, la séquence d'acide nucléique introduite dans ladite cellule par les techniques de l'ADN recombinant et qui code pour un enzyme intervenant dans la voie de biosynthèse de l'oligosaccharide d'intérêt est hétérologue. On entend désigner par séquence d'acide nucléique hétérologue, une séquence d'acide nucléique qui n'est pas présente naturellement dans la cellule selon l'invention. La séquence d'acide nucléique hétérologue selon l'invention peut provenir de tout type cellulaire animal ou végétal, eucaryote ou procaryote et peut provenir de virus.

Parmi les cellules procaryotes à partir desquelles provient la séquence d'acide nucléique hétérologue, il convient de citer les bactéries et notamment *Escherichia coli, Bacillus subtilis, Campylobacter pylori, Helicobacter pylori, Agrobacterium tuméfaciens, Staphylococcus aureus, Thermophilus aquaticus, Azorhizobium caulinodans, Rhizobium leguminosarum, Rhizobium meliloti, Neisseria gonorrhoeae, Neisseria meningitis.*

Parmi les cellules eucaryotes unicellulaires à partir desquelles provient la séquence d'acide nucléique hétérologue il convient de citer les levures et notamment *Saccharomyces cerevisae, Saccharomyces pombe, Candida albicans.*

Selon un mode préféré de réalisation, la séquence d'acide nucléique hétérologue provient de cellules eucaryotes végétales ou animales. Selon un mode encore préféré, la séquence d'acide nucléique hétérologue provient de cellules de mammifères et de préférence de cellules humaines.

Selon un mode préféré de réalisation de l'invention, la cellule selon l'invention est la bactérie *Escherichia coli* et la séquence d'acide nucléique introduite dans la bactérie et codant pour l'enzyme selon l'invention provient de préférence de bactérie choisie dans le groupe cité ci-dessus.

Selon un mode préféré de réalisation de l'invention, la séquence d'acide nucléique codant pour l'enzyme selon l'invention est introduite dans ladite cellule sous la forme d'un vecteur d'expression. Le vecteur doit comporter un promoteur, des signaux d'initiation et de terminaison de la traduction, ainsi que des régions appropriées de régulation de la transcription. Le vecteur doit pouvoir être maintenu de façon stable dans la cellule au cours des générations successives et peut éventuellement posséder des signaux particuliers spécifiant la sécrétion de l'enzyme traduite. Ces différents signaux de contrôle sont choisis en fonction de l'hôte cellulaire utilisé. A cet effet, les séquences d'acides nucléiques peuvent être insérées dans des vecteurs à réplication autonome au sein de l'hôte choisi ou dans des vecteurs intégratifs qui s'intègre dans le génome de l'hôte choisi. De tels vecteurs sont préparés selon les méthodes couramment utilisées par l'homme du métier, et les clones en résultant peuvent être introduits dans un hôte cellulaire approprié par des méthodes standard, telles par exemples le choc thermique ou l'électroporation.

L'invention vise en outre les cellules ci-dessus caractérisées en ce qu'elles sont transformées par au moins un acide nucléique isolé recombinant codant pour l'enzyme selon l'invention ou par au moins un vecteur recombinant tel que défini ci-dessus.

Le procédé selon l'invention se caractérise en ce que ladite modification effectuée par ladite enzyme est choisie parmi la glycosylation, la sulfatation, l'acétylation, la phosphorylation, la succinylation, la méthylation et l'addition d'un groupe énolpyruvate, la sialylation, la fucosylation. Plus particulièrement, le procédé selon l'invention se caractérise en ce que ledit enzyme est un enzyme capable d'effectuer une glycosylation choisi parmi les glycosyl-transférases, les glycosyl-hydrolases, les glycosyl-phosphorylases. Selon un mode préféré de réalisation, l'enzyme capable d'effectuer la glycosylation est une glycosyl-transférase. Selon un mode de réalisation préféré, la glycosyl-transférase selon l'invention est choisie parmi la β-1,3-N-acétyl-glucosaminyl-transférase, la β-1,3 galactosyl-transférase, l'α-1,3 N-acétyl-galactosaminyl-transférase, la β-1,3 glucuronosyl-transférase, la β-1,3 N-acétyl-galactosaminyl-transférase, la β-1,4 N-acétyl-galactosaminyl-transférase, la β-1,4-galactosyl-transferase, l'α-1-3-galactosyl-transférase, l'α-1,4-galactosyl-transférase, l'α-2,3-sialyl-transférase, l'α-2,6 sialyl-transférase, l'α-2-8 sialyl-transférase, l'α-1,3-fucosyl-transférase, l'α-1,4 fucosyl-transférase, l'α-1,2 fucosyl-transférase. Les glycosyl-transférases utilisées dans la présente invention sont capables de la conjugaison stéréospécifique d'unité de saccharides spécifiques activés sur une molécule acceptrice spécifique. Les saccharides activés consistent en général en des dérivés de saccharides uridine-, guanosine- et cytidine-diphosphate. Ainsi, les saccharides activés peuvent être un UDP-saccharide, un GDP-saccharide, un CMP-saccharide.

Certains gènes codant pour des glycosyl-transférases utilisées dans le procédé selon l'invention ont été décrits au préalable ; ainsi la demande internationale de brevet WO 96 10086 décrit la synthèse classique d'oligosaccharide : au cours d'une première étape, les différentes glysosyl-transférases sont produites dans des bactéries recombinantes possédant les gènes *lgtA, lgtB* et *lgtC* de *Neisseria gonorrhoeae ,* puis après purification des enzymes recombinantes ainsi produites, les oligosaccharides sont synthétisés *in vitro* en présence des précurseurs et des nucléotides-sucres nécessaires.

Selon certains modes de réalisation de l'invention, l'enzyme capable d'effectuer une acétylation est codé par le gène NodL de la bactérie *Azorhizobium caulinodans.* Selon un autre mode de réalisation, l'enzyme capable d'effectuer une sulfatation est codé par le gène NodH de la bactérie *Rhizobium meliloti.*

Le procédé selon l'invention est caractérisé en ce que ladite mise en culture cellulaire est effectuée de préférence sur un substrat carboné ; selon un mode particulier de réalisation de l'invention, ledit substrat carboné est choisi parmi le glycérol et le glucose. D'autres substrats carbonés peuvent également être employés ; il convient de citer le maltose, l'amidon, la cellulose, la pectine, la chitine. Selon un autre mode de réalisation, la culture cellulaire est réalisée sur un substrat composé d'acides aminés et/ou de protéine et/ou de lipides.

Le procédé selon l'invention se caractérise en ce que ladite étape de mise en culture est effectuée dans des conditions permettant l'obtention d'une culture à haute densité cellulaire ; cette étape de mise en culture comprend une première phase de croissance cellulaire exponentielle assurée par ledit substrat carboné, une seconde phase de croissance cellulaire limitée par ledit substrat carboné qui est ajouté de manière continue et enfin une troisième phase de croissance cellulaire ralentie obtenue en ajoutant de manière continue dans la culture une quantité dudit substrat diminuée par rapport à la quantité de substrat ajoutée à l'étape b) de façon à augmenter la teneur en oligosaccharides produits dans la culture à haute densité cellulaire.

Le procédé selon l'invention se caractérise en ce que la quantité de substrat ajouté de manière continue dans la culture cellulaire au cours de ladite phase c) est diminuée d'au moins 30%, de préférence 50%, de manière préférée 60% par rapport à la quantité de substrat ajouté de manière continue lors de ladite phase b). Le procédé selon l'invention se caractérise également en ce que ledit précurseur exogène est ajouté lors de la phase b).

Selon un mode de réalisation de l'invention, le procédé se
caractérise en ce que ledit précurseur exogène est de nature glucidique, de préférence de nature oligosaccharidique.

L'originalité et la faisabilité du procédé selon l'invention repose sur l'utilisation de deux modes d'internalisation du précurseur exogène qui ne détruisent l'intégrité de la cellule ni n'atteignent ses fonctions vitales. Ceci exclut notamment les techniques classiques de perméabilisation de la membrane par des solvants organiques qui vont bloquer la croissance et le métabolisme énergétique. Les deux modes possibles d'internalisation du précurseur exogène utilisent un mécanisme de transport passif ou actif.

La présente invention concerne un procédé caractérisé en ce que ledit précurseur est internalisé selon un mécanisme de transport actif. On entend désigner par internalisation par transport actif, l'aptitude des cellules et de manière préférée les bactéries à admettre et concentrer sélectivement certaines substances ou précurseurs exogènes dans leur cytoplasme. Ce transport est réalisé par des transporteurs de nature protéique appelés perméases qui agissent comme des enzymes ; les perméases sont des catalyseurs inductibles, c'est-à-dire synthétisés en présence de substrat ou du précurseur. Selon un mode particulier de réalisation de l'invention, le lactose et les β-galactosides constituent des précurseurs qui sont transportés activement dans le cytoplasme de la bactérie *Escherichia coli* par la lactose perméase encore appelée galactoside perméase. L'invention concerne donc un procédé selon l'invention caractérisé en ce que ledit transport actif dudit précurseur est réalisé par la lactose perméase. Le lactose perméase possède une spécificité assez large qui lui permet de transporter, outre le lactose, d'autres molécules. Elle est en effet capable de transporter divers β-galactosides naturels ou synthétiques, des α-galactosides et le saccharose. C'est donc l'un des objets de l'invention de fournir, selon un mode préféré de réalisation, un procédé caractérisé en ce que ledit précurseur est le lactose qui constitue le motif de base de très nombreux oligosaccharides biologiquement actifs. Il est également dans l'étendue de l'invention de fournir un procédé caractérisé en ce que en ce que ledit précurseur est choisi dans le groupe composé de : (i) β-galactosides naturels ou synthétiques, de préférence dans le 4-O-β-D-galactopyranosyl-D-fructofuranose (lactulose), le 3-O-β-D-galactopyranosyl-D-arabinose et l'allyl-β-D-galactopyranoside, (ii) d'α-galactosides, de préférence le mélibiose et le raffinose, l'allyl-α-D-galactopyranoside (iii) de saccharose.

La specificité de la lactose perméase peut même être modifiée par mutation et permettre le transport d'autres composés tels le maltose et le cellobiose. Tous ces composés peuvent donc être utilisés comme précurseur pour la synthèse d'oligosaccharides. Il est également dans l'étendue de cette invention d'utiliser comme précurseurs des analogues du lactose possédant un groupement chimiquement réactif pour une fonctionalisation ultérieure du produit de préférence un de ces analogues est l'allyl β-D-galactopyranoside. Il est également dans l'étendue de cette invention d'utiliser d'autres perméases modifiées ou non par les techniques de l'ADN recombinant pour permettre l'internalisation de différents types de précurseurs.

Les β-galactosides sont normalement hydrolysés dans le cytoplasme de la bactérie par la β-galactosidase codée par le gène *LacZ.* Afin de s'affranchir de ce problème, un mutant bactérien *lacZ-*dépourvu d'activité β-galactosidase est utilisé lorsque le précurseur employé, est le lactose et/ou un β-galactoside. L'objet de l'invention consiste à fournir un procédé caractérisé en ce que ladite cellule est dépourvue d'activité enzymatique susceptible de dégrader ledit précurseur ainsi que lesdits intermédiaires métaboliques.

Selon un mode particulier, l'invention se rapporte à un procédé décrit ci-dessus caractérisé en ce que ledit précurseur est l'acide sialique. Dans ce cas, ledit transport actif dudit précurseur est réalisé par la perméase NanT.

Selon un autre mode particulier, l'invention porte sur un procédé décrit ci-dessus caractérisé en ce que ledit précurseur est l'acide sialique et le lactose. Dans ce cas, ledit transport actif dudit précurseur est réalisé par la lactose perméase et la perméase NanT.

Dans le procédé selon l'invention, ladite cellule est dépourvue d'activité enzymatique susceptible de dégrader ledit précurseur ou lesdits précurseurs.

Selon un mode préféré de réalisation, le procédé se caractérise en ce que ladite cellule a un génotype choisi parmi *LacZ-* et/ou *NanA-.*

Selon un autre aspect de l'invention, le procédé se caractérise en ce qu'il comprend en outre l'addition d'un inducteur dans ledit milieu de culture pour induire l'expression dans ladite cellule dudit enzyme et/ou d'une protéine impliquée dans ledit transport actif; selon un mode préféré de réalisation, le procédé selon l'invention est caractérisé en ce que ledit inducteur est l'isopropyl β-D-thiogalactoside (IPTG) et ladite protéine est la lactose perméase.

L'invention permet pour la première fois de produire des oligosaccharides complexes avec des rendements de l'ordre du gramme par litre. Selon sa taille, l'oligosaccharide soit s'accumule dans le cytoplasme bactérien, soit est sécrété dans le milieu de culture. Ainsi, selon un mode préféré de réalisation, le procédé selon l'invention est utilisé pour la production du trisaccharide 4-O-[3-O-(2-acétamido-2déoxy-β-D-glucopyranosyl)-β-D-galactopyranosyl]-D-glucopyranose, (β-D-GlcNac-[1→3]-β-D-Gal-[1→4]-D-Glc); il se caractérise en ce que ladite cellule est une bactérie de génotype *LacZ-, LacY*^{*+*}*,* ledit enzyme est la β-1,3-N-acétyl-glucosaminyl-transférase, ledit substrat est le glycérol, ledit inducteur est l'isopropyl β-D-thiogalactoside (IPTG) et ledit précurseur est le lactose.

Selon un deuxième mode préféré de réalisation, le procédé selon l'invention est utilisé pour la production du lacto-N-néotétraose et de polylactosamine ; il se caractérise en ce que ladite cellule est une bactérie de génotype *Lac Z*^{*-*}*, Lac Y*⁺, lesdits enzymes sont la β-1,3-N-acétyl-glucosaminyl-transférase et la β-1,4-galactosyl-transférase, ledit substrat est le glucose, ledit inducteur est l'isopropyl-β-D-thiogalactoside (IPTG), ledit précurseur est le lactose.

Selon un troisième mode préféré de réalisation, le procédé selon l'invention est utilisé pour la production de l'allyl 3-O-(2-acétamido-2déoxy-β-D-glucopyranosyl)-β-D-galactopyranoside, (β-D-GlcNac-[1→3]-β-D-Gal-1→O-allyl); il se caractérise en ce que ladite cellule est une bactérie de génotype *LacZ*^{*-*}*, LacY*^{*+*}*,* ledit enzyme est la β-1,3-N-acétyl-glucosaminyl-transférase, ledit substrat est le glycérol, ledit inducteur est l'isopropyl β-D-thiogalactoside (IPTG), ledit précurseur est l'allyl-β-D-galactopyranoside.

Selon un quatrième mode préféré de réalisation, le procédé selon l'invention est utilisé pour la production d'analogues du lacto-N-néo-tétraose et de polylactosamines dans lesquels le résidu glucose est remplacé par un groupement allyl ; il se caractérise en ce que ladite cellule est une bactérie de génotype *Lac*Z-, *LacY+,* lesdits enzymes sont la β-1,3-N-acéryl-glucosaminyl-transférase et la β-1,4-galactosyl-transférase, ledit substrat est le glucose, ledit inducteur est l'isopropyl β-D-thiogalactoside (IPTG) et ledit précurseur est l'allyl-β-D-galactopyranoside.

Selon un cinquième mode préféré de réalisation, le procédé selon l'invention est utilisé pour production de l'allyl-β-D-lactosamine (β-D-Gal-[1->4]-β-D-GlcNac-1->O-allyl); il se caractérise en ce que ladite cellule est une bactérie de génotype *LacZ-, LacY+ ,* ledit enzyme est la β-1,4-galactosyl-transférase, ledit substrat est le glycérol, ledit précurseur est l'allyl-N-acétyl β-D-glucosaminide (β-D-GlcNac-[1->O-allyl)).

L'invention concerne également un procédé qui permet d'envisager la production d'un grand nombre d'oligosaccharides différents obtenus par glycosylation du lactose. En effet, outre les gènes *lgtA* et *lgtB* qui code respectivement pour la β-1,3-N-acétyl-glucosaminyl-transférase et la β-1,4-galactosyl-transférase, plusieurs gènes de glycosyl-transférases bactériennes utilisant le lactose comme précurseur ont été récemment clonés. Il s'agit de *lgtC* (β-1,4-galactosyl-transférase) et de *Lst* (α-2,3 sialyl-transférase) (Gilbert et *al.,* 1997). L'utilisation de ces gènes dans un procédé selon l'invention permet de produire des molécules comme le globotriose (P^{k} blood antigen) et le sialyl-lactose. Par ailleurs, la coexpression des gènes LgtA et *LgtB* avec le gène de la α-1,3 fucosyl-transférase de *Helicobacter pylori* (Martin et *al.,* 1997) selon un procédé selon l'invention permet l'obtention du Lewis^{x} pentasacharide. L'addition du gène Lst (α-2,3 sialyl-transférase) donne accès au sialyl Lewis^{x} hexasaccharide.

Le procédé selon l'invention permet également d'obtenir un grand nombre d'oligosaccharides différents obtenus par glycosylation de précurseurs exogènes autre que le lactose et transportés par la lactose perméase ou par d'autres perméases.

Le procédé selon l'invention permet d'obtenir un grand nombre d'oligosaccharides différents obtenus par modification (sulfatation, acétylation, phosphorylation, succinylation, méthylation, addition d'un groupement énolpyruvate) *in vivo* de précurseurs. La synthèse de certains oligosaccharides peut nécessiter outre la modification de précurseurs exogènes, la modification de précurseurs endogènes. Ainsi, il est envisageable d'introduire dans une bactérie *Escherichia coli* K12 le gène d'enzyme impliqué dans le métabolisme de précurseur endogène pour permettre la production de certains nucléotides-sucres tels que par exemple le CMP-acide sialique, l'UDP-GalNAc ou le GDP-fucose qui ne sont pas normalement produit par cette souche bactérienne, afin de réaliser la synthèse d'un oligosaccharide d'intérêt. Par exemple, l'UDP-GalNAc peut être produit à partir de l'UDP-GlcNAc si le gène de l'épimérase est introduit dans une cellule selon l'invention.

Contrairement à la méthode enzymatique de synthèse *in vitro* d'oligosaccharides qui nécessite l'utilisation de molécules très onéreuses comme l'ATP, l'acétyl-CoA, le PAPS (adénosine 3' phosphate- 5'phosphosulfate), ou le phospho-énolpyruvate l'un des intérêts de la présente invention réside dans le fait que ces molécules sont naturellement recyclées dans la cellule permettant ainsi d'abaisser les coûts de production des oligosaccharides.

Un autre objet de l'invention concerne un procédé décrit ci-dessus pour la production du 3'-sialyllactose(α-NeuAc-[2→3]-[β-D-Gal-[1→4]-β-D-Glc) ou du 6'-sialyllactose (α-NeuAc-[2→6]-β-D-Gal-[1→4]-β-D-Glc) caractérisé en ce que :
- ladite cellule est une bactérie de génotype *LacZ-, LacY+, NanA-, NanT+ ;*
- lesdits enzymes sont la CMP-NeuAc-synthase et l'α-2,3 sialyl-transférase ou l'α-2,6 sialyl-transférase;
- ledit substrat est le glycérol ;
- ledit inducteur est l'isopropyl-β-D-thiogalactoside (IPTG) ;
- lesdits précurseurs sont le lactose et l'acide sialique.

Selon un sixième mode préféré de réalisation qui complète le deuxième mode décrit ci-dessus, le procédé selon l'invention est utilisé pour la production d'un dérivé sialylé du lacto-N-néotétraose et de polylactosamine (lacto-N-néo-hexaose, lacto-N-néo-octaose, lacto-N-néo-décaose) caractérisé en ce qu'il comprend en outre un dit enzyme choisi parmi l'α-2,3 sialyl-transférase, l'α-2,6 sialyl-transférase, et que ladite cellule a en outre un génotype *NanA-, NanT+* et exprime le gène de la CMP-NeuAc-synthase, lesdits accepteurs sont le lactose et l'acide sialique.

Un autre objet de l'invention concerne un procédé décrit ci-dessus pour la production du lacto-N-néotétraose, du β-D-Gal[1→4]-β-D-GlcNac[1→3]-β-D-Gal[1→4]-α-L-Fuc-[1→3])β-D-Glc, du β-D-Gal-[1→4]-(α-L-Fuc-[1→3])-β-D-GlcNac-[1→3]-β-D-Gal-[1→4]-α-L-Fuc-[1→3])-β-D-Glc, du β-D-Gal-[1→4]-(α-L-Fuc-[1→3])-β-D-GlcNac-[1→3]-β-D-Gal-[1→4]-β,D-GlcNAc-[1→3]-β-D-Gal[1→4]-(α-L-Fuc-[1→3]-β-D-Glc,
caractérisé en ce que
- ladite cellule est une bactérie de génotype *Lac Z*^{*-*}*, Lac Y*^{*+*}*, Wca J*⁻, et surexprime *RcsA ;*
- lesdits enzymes sont la β-1,3-N-acétyl-glucosaminyl-transférase, la β-1,4-galactosyl-transférase, la α-1,3 fucosyl-transférase ;
- ledit substrat est le glucose ;
- ledit inducteur est l'isopropyl-β-D-thiogalactoside (IPTG) ;
- ledit précurseur est le lactose.

Selon un septième mode préféré de réalisation, le procédé selon l'invention est utilisé pour la production de 3'fucosyllactose (β-D-Gal-[1→4]-(α-L-Fuc-[1→3]-D-Glc) ou de 2'fucosyllactose (β-D-Gal-[1→2]-(α-L-Fuc-[1→3]-D-Glc) caractérisé en ce qu'il comprend un dit enzyme choisit parmi l'α-1,3 fucosyltransférase ou l'α-1,2 fucosyltransférase, et que la cellule a un génotype *wcaj*^{*-*} *lacZ*^{*-*} et surexprime le gène *rcsA* et que ledit précurseur est le lactose.

Selon un huitième mode préféré de réalisation, le procédé selon l'invention est utilisé pour la production d'un dérivé fucosylé du lacto-N-néotétraose et de polylactosamine (lacto-N-néo-hexaose, lacto-N-néo-octaose, lacto-N-néo-décaose) caractérisé en ce qu'il comprend en outre un dit enzyme choisi parmi l'α-1,2 fucosyl-transférase, l'α-1,3 fucosyl-transférase, et que ladite cellule a en outre un génotype *Wca J*^{*-*} et surexprime le gène *Rcs A,* ledit accepteur étant le lactose.

Selon un neuvième mode préféré de réalisation, le procédé selon l'invention est utilisé pour la production d'un dérivé sialylé et fucosylé du lacto-N-néotétraose, lacto-N-néo-décaose) caractérisé en ce qu'il comprend en outre un dit enzyme choisi parmi le l'α-2,3 sialyl-transférase, l'α-2,6 sialyl-transférase, et en outre un dit enzyme choisi parmi l'α-1,2 fucosyl-transférase, l'α-1,3 fucosyl-transférase, et que ladite cellule a en outre un génotype *NanA*-, *NanT+, Wca J* et surexprime le gène *Rcs A* et le gène de la CMP-NeuAc-synthase, lesdits accepteurs sont le lactose et l'acide sialique.

Les procédés des modes 1 à 9 évoqués précédemment peuvent être mis en oeuvre pour la production d'analogue d'oligosaccharides dans lesquels le résidu glucose est remplacé par un groupement allyl, ledit précurseur étant l'allyl-β-D galactoside et non plus le lactose.

Un autre objet de l'invention est de fournir un procédé pour produire des oligosaccharides marqués ou enrichis avec des radioisotopes; de tels oligosaccharides sont extrêmement précieux pour les études fondamentales de biologie ou d'analyse conformationnelle. L'invention concerne donc un procédé de production d'oligosaccharide marqué par au moins un radioisotope caractérisé en ce que ladite cellule est cultivée sur ledit substrat carboné marqué par ledit radioisotope et/ou en présence d'undit précurseur marqué par ledit radioisotope. Les radioisotopes sont choisis de préférence dans le groupe composé de : ¹⁴C, ¹³C, ³H, ³⁵S, ³²P, ³³P.

L'intérêt industriel du procédé selon l'invention est évident car il permet pour la première fois d'atteindre une production de l'ordre du kilogramme d'oligosaccharides complexes d'intérêt biologique. Tous les oligosaccharides d'intérêt biologique dont nous envisageons la synthèse à l'échelle industrielle ne sont actuellement disponibles qu'à l'échelle du mg et à des coût extrêmement élevés (jusqu'à 1 million de franc le gramme) ; le prix de revient de ces composés produits par la présente voie microbiologique sont infiniment moindre.

Des caractéristiques et avantages de la présente invention seront mieux mis en évidence à la lecture des exemples et des figures suivantes dont les légendes sont représentées ci-après.

### FIGURES

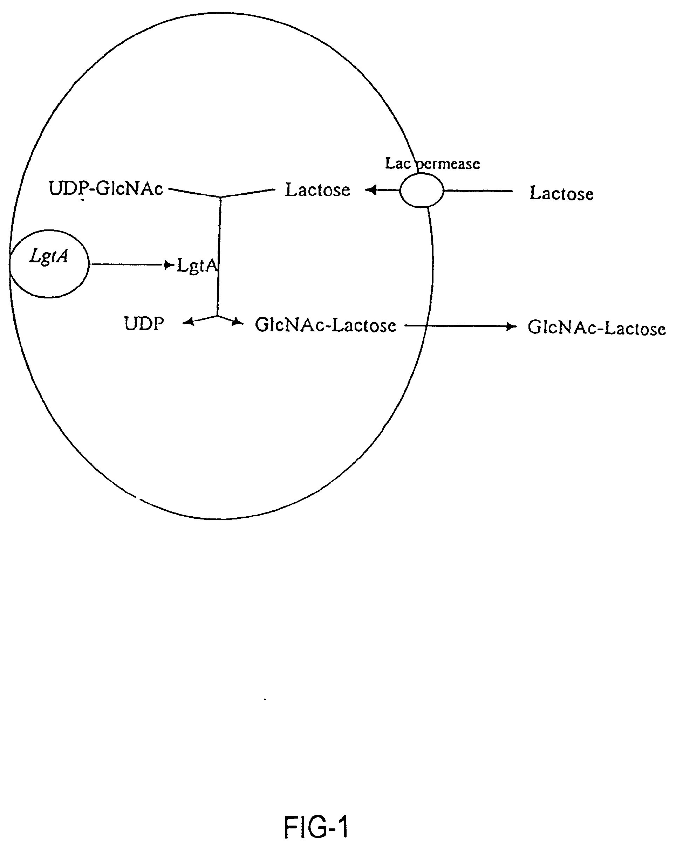

### Figure 1 : Principe du procédé de production du trisaccharide 4-O-[3-O-(2-acétamido-2déoxy-β-D-glucopyranosyl)-β-D-galacto-pyranosyl]-D-glucopyranose, (β-D-GlcNac-[1→3]-β-D-Gal-[1→4]-D-Glc)

Le lactose( β-D-Gal-[1-4]-β-D-Glc) est transporté dans la cellule par la lactose perméase (Lac perméase). Le lactose ne peut pas être hydrolysé dans la cellule car la souche est un mutant *LacZ-.* L'expression du gène *lgtA* permet la production de l'enzyme LgtA qui transfère un GlcNAc de l'UDP-GlcNAc sur une molécule de lactose. Le trisacharide formé (β-D-GlcNAc-[1-3]-β-D-Gal-[1-4]-β-D-Glc) est excrété dans le milieu.

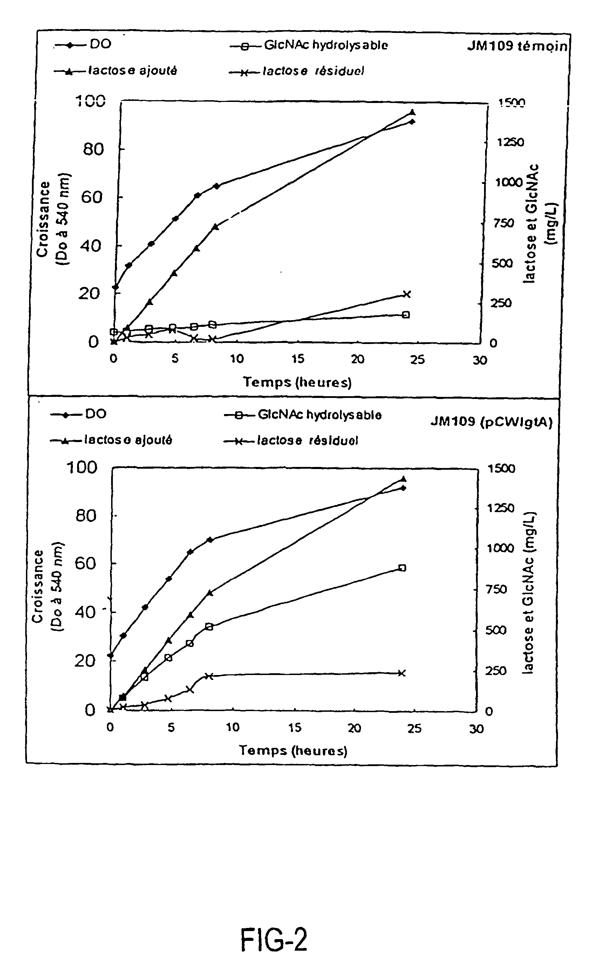

### Figure 2 : Culture à haute densité cellulaire de la souche JM109 témoin et de la souche JM109 (pCW1gtA) possédant le gène de la glycosyle transférase LgtA.

Le lactose est ajouté en continu et le lactose résiduel est déterminé enzymatiquement. La concentration de GlcNAc hydrolysable dans le milieu de culture est mesurée colorimétriquement après hydrolyse acide. Le lactose ajouté représente la quantité totale cumulée de lactose qui a été ajouté en continu.

### Figure 3: Spectre de masse en mode FAB⁺ du trisaccharide 4-O-[3-O-(2-acétamido-2déoxy-β-D-glucopyranosyl)-β-D-galactopyranosyl]-D-glucopyranose, (β-D-GlcNac-[1→3]-β-D-Gal-[1→4]-D-Glc) purifiée du surnageant de culture de la souche JM109(lgtA).

On observe les deux ions quasi-moléculaires [M+H]⁺ et [M+Na]⁺ à m/z 546 et 568. On observe également un ion [M+H]⁺ à m/z 442 qui est du à la présence de β-D-GlcNAc-[1-3]-IPTG. Ceci indique que l'IPTG (isopropyl β-D-thiogalactose) utilisé pour induire la Lac perméase et LgtA est également glycosylé.

### Figure 4 : Spectre du trisaccharide 4-O-[3-O-(2-acétamido-2déoxy-β-D-glucopyranosyl)-β-D-galactopyranosyl]-D-glucopyranose, (β-D-GlcNac-[1→3]-β-D-Gal-[1→4]-D-Glc) en RMN du proton à 323°K.

Le signal à 1,4 ppm est du aux protons du groupement isopropyl du dérivé glycosylé de l'IPTG.

### Figure 5 : Spectre RMN ¹³C du trisaccharide 4-O-[3-0-(2-acétamido-2déoxy-β-D-glucopyranosyl)-β-D-galactopyranosyl]-D-glucopyranose, (β-D-GlcNac-[1→3]-β-D-Gal-[1→4]-D-Glc).

### Figure 6: Principe du procédé de production du lacto-N-néo-tétraose β-D-Gal-[1-4]-β-D-GlcNAc-[1-3]-β-D-Gal-[1-4]-β-D-Glc).

Le lactose (β-D-Gal-[1-4]-β-D-Glc) est transporté dans la cellule par la Lac perméase. Le lactose ne peut pas être hydrolysé dans la cellule car la souche est un mutant *LacZ-.* L'expression du gène *lgtA* permet la production de l'enzyme LgtA qui transfère un GlcNAc de l'UDP-GlcNAc sur une molécule de lactose. Le trisaccharide formé est ensuite utilisé comme précurseur par LgtB qui transfère une molécule de galactose de l'UDP-Gal pour former le lacto-N-néo-tétraose (β-D-Gal-[1-4]-β-D-GlcNAc-[1-3]-β-D-Gal-[1-4]-β-D-Glc) .

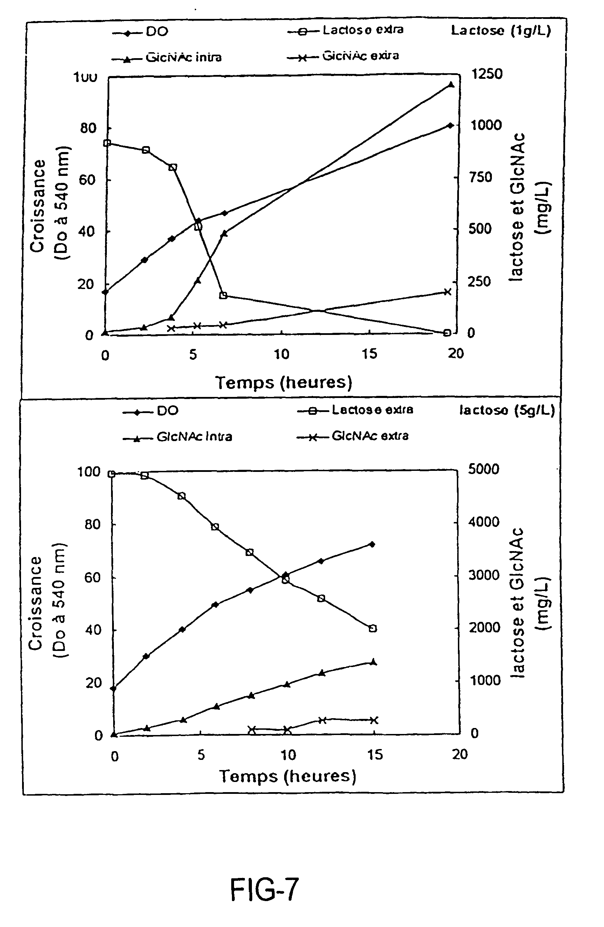

### Figure 7 : Culture à haute densité cellulaire de la souche JM109 /pCWlgtA, pBBlgtB).

Culture en présence de lactose à forte (5 g.l⁻¹) et à faible concentration (1 g.l⁻¹).

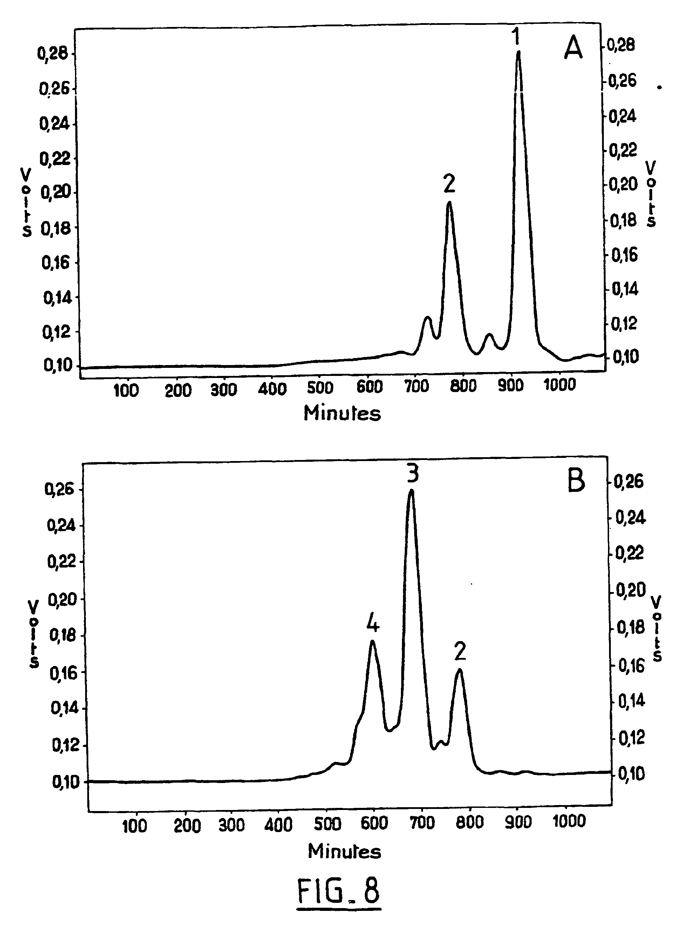

### Figure 8 : Séparation sur Biogel P4 des oligosaccharides produits par la souche JM109 (pCWlgtA, pBBlgtB) en présence de lactose à une concentration initiale de 5 g.l⁻¹ (A) ou de 1 g.l⁻¹ (B).

Les pics 1, 2, 3, 4 correspondent respectivement aux lacto-*N-*néo-tétraose, lacto-*N*-néo-hexaose, lacto-*N* néo-octaose et lacto-*N-*néo-décaose.

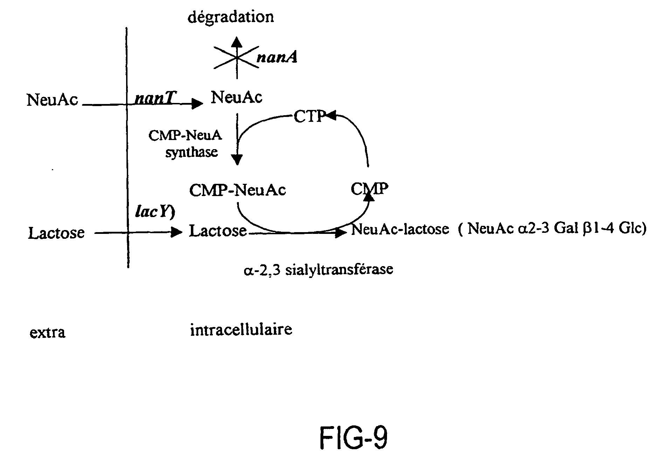

### Figure 9 : Principe du procédé de production du sialyllactose

Le lactose et l'acide sialique (NeuAc) sont internalisés dans la cellule par la lactose permease *(lacY)* et la permease de l'acide sialique *(nanT).* Ces deux composés ne sont pas dégradés dans la cellule car la souche est un mutant *lacZ-* et *nanA- .* L'expression de la CMP-NeuA synthase et de l'α-2,3 sialyltransferase permet l'activation de l'acide sialique internalisé en CMP-NeuAc et son transfert sur du lactose intracellulaire.

### EXEMPLES

### Exemple 1 : Matériels et méthodes

### 1.1. Origine des plasmides et souches bactériennes

Les souches JM 107 et JM 109 d*'Escherichia coli* K12 (Yannisch-Perron et al 1984) ont été utilisées comme cellules hôte pour tous les exemples de production d'oligosaccharides décrits. Les souches ont été obtenues de la DSM (Deutsche Sammlung von Mikroorganismen) Le génotype de la souche JM109 est le suivant : *F traD36 lacl*^{*q*} *Δ(lacZ)M15 proA*^{*+*}*B*^{*+*}/*e14*^{*-*}*(McrA*^{*-*}*) Δ(lac-proAB) supE44 recA1 endA1 gyrA96 (Nal*^{*r*}*) thi hsdR17 relA1.* Le génotype de la souche JM107 est identique à celui de la souche JM109 excepté le fait que le gène *recA1* n'est pas inactivé.

Les gènes *lgtA* et *lgtB* de *Neisseria meningitis* MC58 ont été fournis par Dr W. Wakarchuk (Institute for Biological Sciences, National Research council of Canada, 100 Sussex Drive, Ottawa, Ontario, K1A OR6, Canada) sous la forme de deux plasmides pCW, l'un contenant le gène *lgtA* (nommé ici pCWlgtA) et l'autre contenant le gène *lgtB* (nommé ici pCWlgtB). Les séquences de ces deux gènes sont disponibles dans la banque de données GenBank sous le n° U25839. Le plasmide pLitmus28 a été acheté à la société New Englands Biolabs. Le plasmide pBBR1MCS a été fourni par le Dr M. Kovach (Department of Microbiology and Immunology, Louisiana State University, Shreveport, LA 71130-3932, USA.)

Les gènes de la CMP-sialic acid synthase et de l'α-2,3 sialyltransferase de *Neisseria meningitis* MC58 ont été fournis par le Dr M Gilbert (Institute for Biological Sciences, National Research council of Canada, 100 Sussex Drive, Ottawa, Ontario, K1A OR6, Canada) sous la forme de deux plasmides NSY-01 et NST-01. Le plasmide NSY-01 est un dérivé du plasmide pT7-7 qui contient le gène (GenBank U60146) de la CMP-sialic acid synthase (Gilbert et al 1997). Le plasmide NST-01 est un dérivé du plasmide pBluescript Sk- qui contient le gène (GenBank n° U60660) de l'α-2,3 sialyltransferase (Gilbert et al. 1996)

Le gène *fucT* de l'α-1,3 fucosyltransferase *d'Helicobacter pylori* a été fourni par le Dr S. Martin (Glaxo Wellcome Research and Development, Gunnels Wood Road, Stevenage, Hertfordshire, SG1 2NY,UK) sous la forme d'un plasmide pHP0651 dérivé du pET-21a. La sequence est disponible à la Genbank (AE000578, gène HP0651).

### 1.2. Sous-clonages.

Nous avons utilisé les techniques standards de biologie moléculaire décrites par Sambrook et al. (1989).

***construction* du plasmide *pBBlgtB :*** Le fragment d'ADN de 0,835 kb contenant le gène *lgtB* a été obtenu par digestion du plasmide pCWlgtB par *BamHI* et *Hin*dIII. Ce fragment a été sous-cloné dans le vecteur pLitmus28 préalablement digéré par *BamH*1 et *Hind*III pour former le plasmide pLitlgtB. Le fragment de 0,9 kb contenant le gène *LgtB* a été excisé du plasmide pLitlgtB par une digestion avec *Xho*I et *Hind*III et sous cloné dans le plasmide pBBR1MCS préalablement digéré par *Xho*I et *Hind*III pour former le plasmide pBBlgtB.

***construction du plasmide pBBnsy*** :Le fragment contenant le gène de la CMP-sialic acid synthase a été excisé du plasmide NSY-01 par une digestion avec *XbaI* et sous cloné dans le plasmide pBBR1MCS préalablement digéré par *XbaI* pour former le plasmide pBBnsy.

***construction du plasmide pBBLnt:*** le gène *lgtA* présent dans la construction pCWlgtA (Gilbert *et al*.) a été amplifié par PCR en même temps que le promoteur UV5 tactac du plasmide à l'aide des amorces CTTTAAGCTTCCGGCTCGTATAA (sens, amont promoteur) et GACAGCTTATCATCGATAAGCTT (antisens, fin IgtA) contenant toutes deux un site *Hind*III. Le fragment amplifié de 1,3kb a ensuite été sous-cloné dans le site *Hind*III du vecteur pBBlgtB.

***construction du plasmide pBBLntRcsA:*** Le gène *rcsA* (Stout et al., 1991) a d'abord été amplifié par PCR à partir d'ADN génomique de JM109 avec les amorces AGGGTACCCATGTTGTTCCGTTTAG (site *Kpn*I*,* gauche *rcsA)* et AATCTAGAGTAATCTTATTCAGCCTG (site *Xba*I*,* droite *rcsA),* puis cloné dans les sites *Kpn*I*-Xba*I du vecteur pBBR1-MCS. Le vecteur pBBR1-MCS-rcsA a alors été ouvert en amont du gène par digestion avec *Kpn*I*,* blunté (kit Amersham), libéré par *Xba*I*,* et inséré dans les sites *Sma*I*-Xba*I de la construction pBBLnt, permettant un clonage en aval de l'ensemble *lgtB*-UV5tactac-*lgtA*, plaçant *rcsA* sous le contrôle du promoteur UV5 tactac.

### 1.3. Conditions de culture

Les cultures de routine et la préparation des inocula furent réalisées sur le milieu LB (Sambrook et al. 1989). Les cultures à haute densité cellulaire ont été réalisées dans un fermenteur de 2 litres contenant un volume initial de 1 litre de milieu ayant la composition suivante : glycérol (17.5 g.l⁻¹) ou glucose (15 g.l -1), NH₄H₂PO₄ (7 g.l⁻¹), KH₂PO₄ (7 g.l⁻¹), MgSO₄.7H₂O (1 g.l⁻¹), thiamine.HCl (4.5 mg.l⁻¹), solution d'oligo-éléments (7.5 ml.l⁻¹), acide citrique (0.5 g.l⁻¹), KOH (2 g.l⁻¹). Le MgSO₄ est autoclavé séparément et la thiamine est stérilisée par filtration. La solution d'oligo-éléments contient : nitrilotriacétate (70 mM, pH 6.5), citrate ferrique (7.5 g.l⁻¹), MnCl₂. 4H₂O (1.3 g.l⁻¹), CoCl₂ 6H₂O (0.21 g.l⁻¹), CuCl₂.2H₂O (0.13 g.l⁻¹), H₃BO₃ (0.25 g.l⁻¹), ZnSO₄. 7H₂O (1.2 g.l⁻¹), Na₂MoO₄.2H₂O (0.15 g.l⁻¹). Les antibiotiques, ampicilline (50 mg.l⁻¹) et chloramphénicol (25 mg.l⁻¹) sont ajoutés pour s'assurer de la présence des différents plasmides. La solution d'alimentation contient du glycérol (500 g.l⁻¹) ou du glucose (400 g.l⁻¹), du MgSO₄.7H₂O (12 g.l⁻¹) et de la solution d'oligo-éléments (25 ml.l⁻¹).

Les cultures à haute densité cellulaire sont inoculées à 2%. Durant tout la culture, le taux d'oxygène dissous est maintenu à 20% de saturation en réglant manuellement le débit d'air et en ajustant automatiquement la vitesse d'agitation. Le pH est régulé automatiquement à 6,8 par l'addition d'ammoniaque aqueux (15% p/v). La température est maintenue à 34°C pour la souche JM109(pCWlgtA) et à 28°C pour la souche JM109(pCWlgtA, pBBlgtB). La stratégie de culture à haute densité comprend généralement 3 phases : une première phase de croissance exponentielle qui est assurée par le substrat carboné (glycérol ou glucose) initialement présent dans le milieu ; une deuxième phase qui débute lorsque la croissance devient limitée par la source de carbone qui est alors ajoutée en continu à un taux de 4,5 g.h⁻¹.l⁻¹ de giycéroi ou 3,6 g.h⁻¹.l⁻¹ de glucose. Dans une troisième phase, ce taux est réduit de 60 % pour ralentir la croissance de manière a augmenter la teneur en oligosaccharides.

### 1.4. Dosage des oligosaccharides

Les échantillons (1 ml) sont prélevés durant la culture et immédiatement centrifugés dans des microtubes. Le surnageant est conservé pour le dosage des oligosaccharides extracellulaires. Le culot bactérien est resuspendu dans 1 ml d'eau puis est incubé dans un bain-marie à 100°C pendant 30 min pour faire éclater les cellules. Après une seconde centrifugation le surnageant est conservé pour le dosage des oligosaccharides intracellulaires.

La concentration de lactose est mesurée en utilisant un kit de détermination enzymatique (Roche diagnostic). Les résidus N-acétyl-glucosamine présents dans les oligosaccharides sont libérés par hydrolyse acide comme précédemment décrit (Samain *et al.,* 1997) et ensuite quantifiés colorimétriquement par la méthode de Reissig et *al.,* (1955); dans la description, on entend par GlcNAc hydrolysable, la quantité de GlcNAc dosée de cette manière.

Le dosage du lactose avec et sans traitement par une neuraminidase permet d'estimer la concentration en sialyl-lactose.

Le fucose total est mesuré colorimétriquement par la méthode au chlorhydrate de cystéine de Dische et Shettles (1948).

### 1.5. Purification des oligosaccharides

A la fin de la culture, les cellules bactériennes sont récoltées par centrifugation. Le surnageant est conservé pour la purification des oligosaccharides extracellulaires. Les cellules bactériennes sont resuspendues dans 1 litre d'eau, puis sont perméabilisées par un traitement thermique (30 min à 100°C) pour libérer les oligosaccharides intracellulaires. Après une deuxième centrifugation ces oligosaccharides sont récupérés dans le surnageant.

Le premier et le deuxième surnageant contenant respectivement les oligosaccharides extra- et intracellulaires sont adsorbés sur charbon actif (100 g par litre de surnageant). Après rinçage à l'eau distillée, les oligosaccharides sont élués avec de l'éthanol à 50% (v/v), concentrés par évaporation et lyophilisés.

Les oligosaccharides sont séparés par chromatographie d'exclusion stérique sur une colonne (4.5 cm x 95 cm) de Biogel P4 permettant l'injection d'environ 300 mg de mélange d'oligosaccharides. L'élution est réalisée avec de l'eau distillée avec un débit de 40 ml.h⁻¹.

Les oligosaccharides non fucosylés sont séparés par chromatographie d'exclusion stérique sur une colonne (4.5 cm x 95 cm) de Biogel P4 permettant l'injection d'environ 300 mg de mélange d'oligosaccharides. L'élution est réalisée avec de l'eau distillée avec un débit de 40 ml.h-¹

Les oligosaccharides fucosylés sont séparés par chromatographie d'exclusion stérique sur une colonne (1,5 cm x 200 cm) de Biogel P2 thermostatée à 60 °C permettant l'injection d'environ 30 mg de mélange d'oligosaccharides. L'élution est réalisée avec de l'eau distillée avec un débit de 30 ml.h⁻¹

Le sialyllactose est séparé des oligosacchariodes neutres par fixation sur une résine Dowex 1X4-400 (sous forme HCO₃⁻). et élué avec un gradient de NaHCO₃ (0 à 100mM). Le bicarbonnate est ensuite éliminé en traitant l'éluat par une résine Dowex 50X4-400 sous forme H⁺.

### 1.6. Préparation des allyl β-D-glucosides

L'allyl β-D-galactopyranoside et l'allyl-N-acétyl-β-D-glucosaminide ont été synthétisés suivant le protocole décrit par Lee et Lee (1974)

### 1.7. Identification et caractérisation structurale des oligosaccharides

Les spectres de masse ont été réalisés avec un spectromètre de masse (Nermag R-1010C). Pour chaque expérience le volume initial de matrice est de 4 µl. Les produits ont été analysés en mode FAB⁺.

Les spectres de RMN ont été obtenus avec un spectromètre Brucker AC300.

### 1.8 Construction de la souche JM107-nanA-

Une souche JM107 incapable de métaboliser l'acide sialique a été préparée par inactivation insertionnelle du gène *nanA* (opéron Nan) codant pour la NeuAc aldolase (Plumbridge et al., 1999). Deux réactions d'amplification par PCR furent réalisées de part et d'autre du centre du gène nanA de façon à y insérer un site de restriction *Bam*HI.

Un premier fragment *Bum*HI-*Xba*I de 1,6 kb comprenant la partie droite de *nan*A a été amplifié à partir d'ADN génomique de JM109 en utilisant les amorces AAAGGATCCAAGATCAGGATGTTCACG et GCTCTAGAATGGTAATGATGAGGCAC et cloné entre les sites *Bam*HI et *Xba*I du vecteur pUC19, formant le vecteur pUC-nan1,6. Un deuxième fragment *Kpn*I*-Bam*HI de 2,1 kb comprenant la partie gauche de nanA a été amplifié en utilisant les amorces AAAGGATCCGCGTAGGTGCGCTGAAAC et AAAGGTACCTCAGGCCACCGTTAGCAG et cloné entre les sites *Kpn*I et *Bam*HI du vecteur pUC-nan 1,6 formant le vecteur pUC-nan-3,7. Le gène de résistance à la kanamycine (pUC-4K, cassette Pharmacia) a ensuite été cloné dans le site *Bam*HI de pUC-nan-3,7. Le fragment de 4,9 kb SacI-XbaI contenant nanA::kan a été inséré dans les mêmes sites du vecteur suicide pCVD442 (Donnenberg et Kaper 1991). Ce plasmide a été utilisé pour obtenir par recombinaison homologue des mutants JM107 *nanA::kan,* sélectionnés pour leur résistance à la kanamycine et leur incapacité à métaboliser l'acide sialique (souche JM107-nanA-).

### 1.9 Construction de la souche JM107col-DE3

La suppression de la capacité à synthétiser l'acide colanique a été réalisée par inactivation insertionnelle du gene *wcaJ* codant pour une glucosyltransférase (Stevenson et al 1996). Un fragment d'ADN de 1,8 kb contenant le gène *wcaJ* et de l'ADN adjacent ont été amplifiés par PCR à partir d'ADN génomique de JM109 et insérés dans un vecteur pTOPO2.1 (kit de clonage PCR Invitrogen), à l'aide des amorces CCACGATCCACGTCTCTCC (droite *wcaJ)* et AAGCTCATATCAATATGCCGCT (gauche *wcaJ).* Il a ensuite été transféré dans un vecteur pUC19 au niveau du site *Eco*RI. Le vecteur ainsi obtenu a été soumis à un traitement par une *Eco*RI méthylase, permettant l'addition subséquente du gène de résistance à la kanamycine dans le site Apol présent au centre de *wcaJ.* L'ADN recombinant *wcaJ::kan* a enfin été transféré dans le vecteur suicide pCVD442 permettant par recombinaison homologue l'obtention de mutants génomiques JM107 contenant le gène inactivé, sélectionnés par PCR à l'aide des amorces ayant servi au clonage (souche JM107-col⁻).

La souche JM107-col- a été rendue lysogène pour le phage λDE3 en utilisant le kit de lysogénisation de Novagen.

### Exemple 2 : Production du trisaccharide 4-O-[3-O-(2-acétamido-2déoxy-β-D-glucopyranosyl)-β-D-galactopyranosyl]-D-glucopyranose, (β-D-GlcNac-[1→3]-β-D-Gal-[1→4]-D-Glc).

Le principe est illustré par la figure 1. Nous avons utilisé la souche JM 109 *d'Escherichia coli* K12 dans laquelle nous avons introduit le plasmide pCWlgtA gène *lgtA*. La souche JM109 est *lacZ-,* c'est à dire qu'elle est incapable d'hydrolyser le lactose. Par contre elle est *lacY*+ *,* ce qui signifie qu'elle peut synthétiser la lactose perméase. Le gène *lgtA* code pour une β-1,3-*N*-acétyl-glucosaminyl-transférase (LgtA) transférant une unité N-acétyl-glucosamine sur le galactose du lactose.

La souche JM109 (pCWlgtA) ainsi que la souche témoin JM109 ont été cultivées à haute densité cellulaire (Samain *et al.,* 1997) sur glycérol comme source de carbone et d'énergie. Après une première phase de croissance exponentielle assurée par le glycérol initialement présent dans le milieu (17,5 g/l), la croissance devient limitée par le glycérol qui est alors ajouté en continu à un taux de 4,5 g.h⁻¹.l⁻¹. Durant cette deuxième phase de la culture, on introduit en continu 90 mg.h⁻¹.l⁻¹ de lactose. On injecte également au début de cette phase de l'IPTG (isopropyl-β-D-thiogalactoside) (0.5 mM) pour induire l'expression de la lactose perméase et de la β-1,3-*N*-acétyl-glucosaminyl-transférase. Comme décrit dans la figure 2, le lactose ajouté ne s'accumule pratiquement pas dans le milieu, indiquant que le lactose est bien internalisé par les cellules bactériennes. On observe avec la souche JM 109 (pCWIgtA) une accumulation importante dans le milieu de culture d'un composé contenant de la N-acétylglucosamine (GlcNAc hydrolysable). La quantité de GlcNAc hydrolysable (3,8 mmole/l) produite correspond presque stoechiométriquement à la quantité de lactose consommé (3,5 mmole/l) suggérant que la totalité du lactose internalisé a été glycosylée par LgtA.

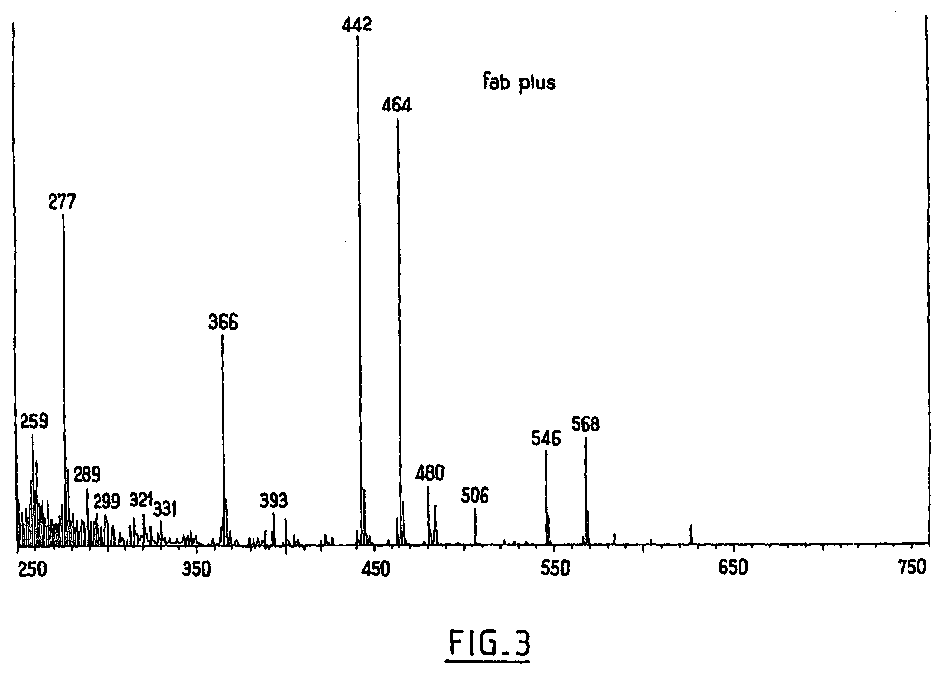

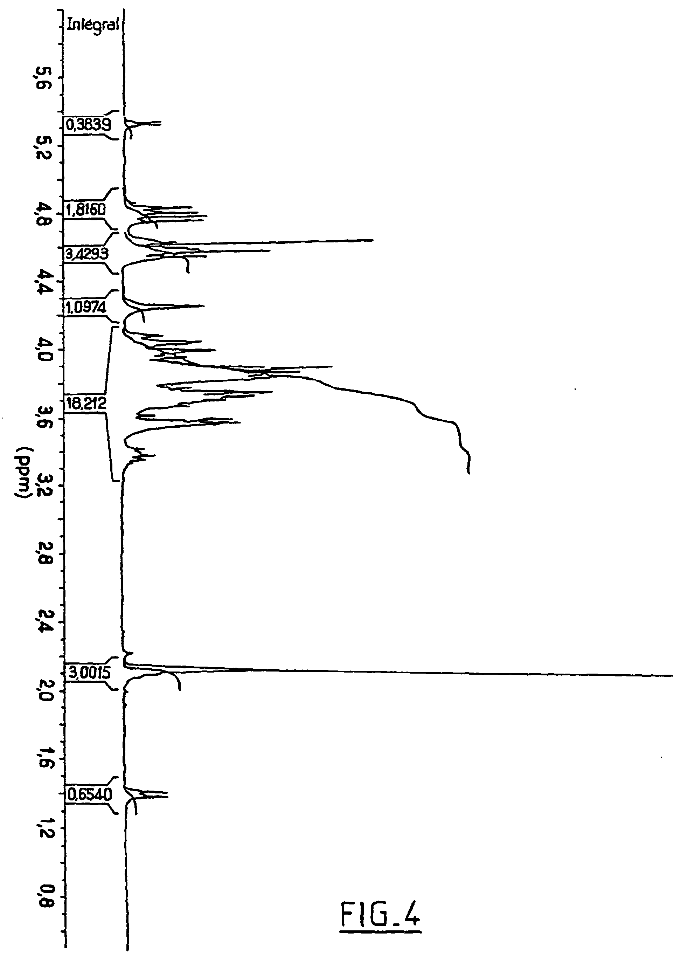

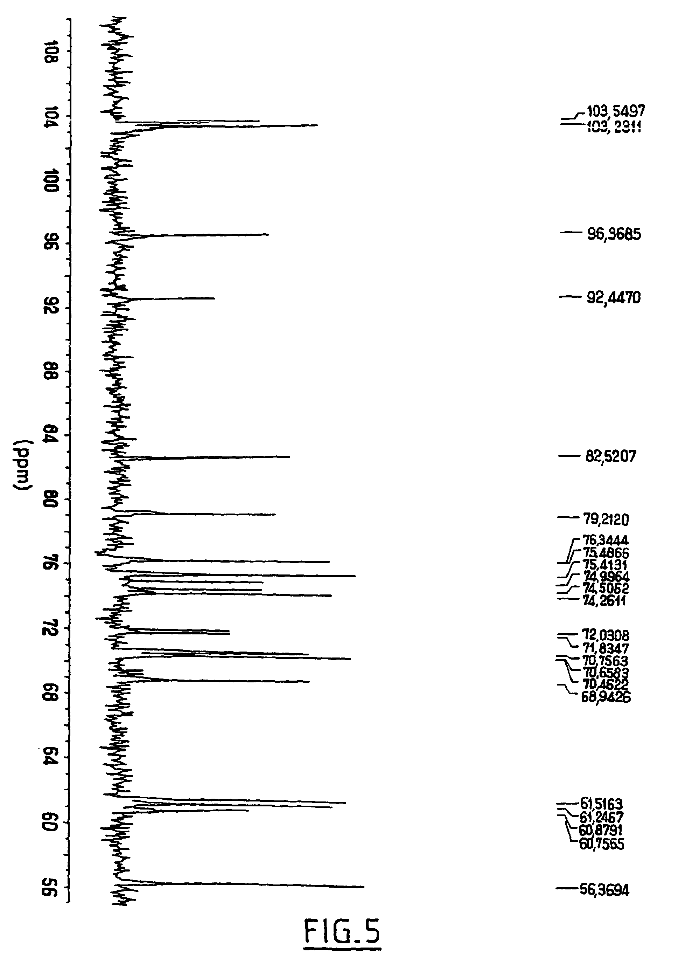

A la fin de la culture, les cellules sont éliminées par centrifugation et les oligosaccharides présents dans le surnageant sont purifiés par adsorption sur charbon actif et élution à l'éthanol. Les oligosaccharides présents sont ensuite séparés suivant leur poids moléculaire sur une colonne de Biogel P4. Un seul composé majoritaire est retrouvé. Les données de spectrométrie de masse et de RMN indique que ce composé est bien le trisaccharide (β-D-GlcNAc-[1->3]-β-D-Gal-[1->4]-β-D-Glc) formé par l'addition d'un résidu GlcNAc sur une molécule de lactose. Le spectre de masse en mode FAB⁺ montre en effet la présence d'un ion quasi-moléculaire [M+H]⁺ à m/z 546 (figure 3). Le spectre RMN du ¹H confirme la structure trisaccharidique, la présence d'un groupement acétyl et la configuration β des deux liaisons O-glycosidiques (figure 4). Le spectre RMN du ¹³C précise également que la liaison entre la GlcNAc et le galactose est bien de type 1,3 (figure 5).

### Exemple 3: Production de lacto-N-néo-tétraose et de polylactosamine

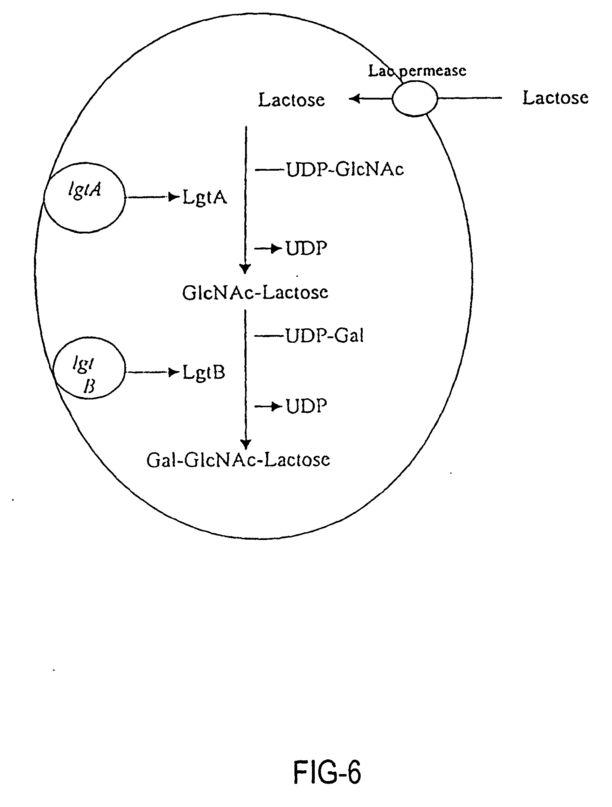

Le principe est décrit sur la figure 6. La souche d'*E. coli* JM 109 a été cotransformée avec les deux plasmides pCWlgtA et pBBlgtB portant respectivement les gènes *lgtA* (utilisé précédemment) et *lgtB* (codant pour une β-1,4-Galactosyl-transférase appelé LgtB). La souche JM109 (pCWlgtA,pBBlgtb) a été cultivée à haute densité cellulaire en utilisant le glucose comme substrat de croissance. Au début de la deuxième phase on ajoute du lactose à forte (5 g.l⁻¹) ou à faible concentration (1 g.l⁻¹) et de l'IPTG à 0.1 mM. Contrairement à ce qui avait été observé avec la souche JM109 (pCWlgtA), on ne détecte, lors de la culture de cette souche, qu'une faible libération de GlcNAc hydrolysable dans le milieu. En revanche, on retrouve de la GlcNAc hydrolysable en quantité importante dans la bactérie (figure 7). Lorsque l'apport de lactose est de 1 g.l⁻¹, on observe une internalisation complète du lactose (2,9 mmole.l⁻¹) et une production totale de GlcNAc lié de 1,45 g.l⁻¹ (6,5 mmole.l⁻¹) soit l'incorporation de plus de deux molécules de GlcNAc par molécule de lactose acceptrice. Quand le lactose est ajouté à forte concentration, l'internalisation est incomplète (3 g.l⁻¹ soit 8.7 mmole.l⁻¹) avec une production de GlcNAc également d'environ 6,5 mmole⁻¹ . Dans ce cas le rapport molaire GlcNAc / lactose est proche de 1, ce qui est cohérent avec la synthèse de lacto-N-néo-tétraose.

La purification de la fraction oligosaccharidique intracellulaire a permis d'obtenir plusieurs composés principaux qui sont bien séparés par chromatograhie sur Biogel P4. Les données de spectrométrie de masse et de RMN indiquent que ces composés correspondent aux structures suivantes : lacto-*N*-néotétraose [M+H]⁺ = 708 ; lacto-*N*-néo-hexaose [M+H]⁺ = 708 ; lacto-N-néo-octaose, [M+Na]⁺ = 1460 et probablement lacto-*N*-néo-décaose. Les proportions respectives de ces différents composés dépendent de la quantité de lactose ajoutée . Ainsi avec 5 g.l⁻¹ de lactose le produit majoritaire est le lacto-*N*-néo-tetraose (figure 8A ). Par contre un apport de lactose plus faible (1 g.l⁻¹) favorise la formation de composés de plus haut degré de polymérisation, le lacto-N-néo-octaose devenant majoritaire (figure 8B).

La formation de polylactosamines homologues supérieurs du lacto-*N*-néo-tétraose s'explique par le fait que LgtA est capable d'utiliser le lacto-*N*-néo-tétraose pour former un pentasaccharide intermédiaire qui est glycosylé par LgtB pour donner du lacto-N-néo-hexaose. Ce dernier est lui même précurseur pour un nouveau cycle de glycosylation aboutissant à la formation de lacto-N-néo-octaose et ainsi de suite jusqu'au lacto-N-néo-décaose.

On n'observe pas la formation significative d'oligosaccharides à nombre de résidus impair et portant un galactose en position terminale non réductrice. Ceci indique que l'élongation des molécules est limitée par l'incorporation du GlcNAc par LgtA et non pas par la galactosylation catalysée par LgtB.

### Exemple 4: Production de l'allyl 3-O-(2-acétamido-2déoxy-β-D-glucopyranosyl)-β-D-galactopyranoside, (β-D-GlcNAc-[1→3]- β-D-Gal-1→O-allyl)

La souche JM109(pCW1gtA) a été cultivée à haute densité cellulaire sur glycérol. Au début de la deuxième phase de culture, on ajoute 0,75 g.l⁻¹ d'allyl-β-D-galactopyranoside et 0,1 mM d'IPTG. On observe une internalisation totale de l'allyl-β-D-galactopyranoside au bout de 9 h avec une apparition stoechiométrique de GlcNAc hydrolysable dans le milieu extracellulaire. Les oligosaccharides présents dans le milieu extracellulaire sont purifiés comme dans l'exemple 2. Le spectre de masse en mode FAB⁺ du produit majoritaire obtenu montre la présence d'un ion quasi-moléculaire [M+H]⁺ à m/z 424 correspondant à la structure β-D-GlcNAc-[1→3]-β-D-Gal-A→O-allyl.

### Exemple 5 : Production du β-D-Gal-[1->4]-β-D-GlcNAc-1->O-allyl

La souche JM109 (pBBlgtB) a été cultivée à haute densité cellulaire sur glycérol. Au début de la deuxième phase de culture, on ajoute 0,5 g.l⁻¹ d'allyl-*N*-acétyl-β-D-glucosaminide (β-D-GlcNAc-1->Oallyl). On observe pendant les 5 premières heures une diminution d'environ 30% de la quantité de GlcNAc hydrolysable extracellulaire, ce qui démontre une internalisation partielle d'allyl-*N*-acétyl-β-D-glucosaminide. Parallèlement on observe une production intracellulaire presque stoechiométrique de GlcNAc hydrolysable et de résidus galactose liés en β (hydrolysables par la β-galactosidase). Ces résultats démontrent que 30% de l'allyl-N-acétyl-β-D-glucosaminide initialement ajouté a été galactosylé par l'activité encodée par le gène *lgtB.* Après purification, la structure du composé attendu (β-D-Gal-[1→4]-β-D-GlcNAc-1→O-allyl) a été confirmée par spectrométrie de masse et RMN.

### Exemple 6 : Production d'analogues du lacto-N-néo-tétraose et de polylactosamines dans lesquels le résidu glucose est remplacé par un groupement allyl

La souche JM 109 (pCWlgtA et pBBlgtB) a été cultivée comme dans l'exemple 3 sauf que l'apport de lactose a été remplacé par l'addition de 0,65 g.l⁻¹ d'allyl-β-D-galactopyranoside. Après purification selon le protocole de l'exemple 3, on obtient 3 composés principaux. Les données de spectrométrie de masse indiquent que ces trois composés correspondent aux tri-, penta- et heptasaccharides suivants :
β-D-Gal-[1->4]-β-D-GlcNAc-[1->3]-β-D-Gal-1->O-allyl, [M+H] = 586;
β-D-Gal-[1->4]-β-D-GlcNAc-[1->3]-β-D-Gal-[1->4]-β-D-GlcNAc-[1->3]-β-D-Gal-1->O-allyl, [M+H] = 951;
β-D-Gal-[1->4]-β-D-GlcNAc-[1->3]-β-D-Gal-[1->4]-β-D-GlcNAc-[1->3]-β-D-Gal-[1->4]-β-D-GlcNAc-[1->3]-β-D-Gal-1->O-allyl, [M+H] = 1316.

### Exemple 7 : Production du 3'-sialyllactose (α-NeuAc-[2→3]-β-D-Gal-[1>-4]-β-D-Glc)

Le principe est illustré par la figure 9. Les gènes de biosynthèse de l'acide sialique et du CMP-NeuAc ne sont pas présents chez *E*. *coli* K12. Par contre *E*. *coli* K12 est capable de dégrader l'acide sialique ( Plumbridge et. Vimr 1999) et possède une perméase (NanT) qui permet de faire pénétrer de l'acide sialique exogène dans la cellule. Cet acide sialique est ensuite normalement catabolisé par une aldolase (NanA).

Nous avons utilisé la souche d'*Escherichia coli* K12 JM107-nanA- (exemple 1) et une souche témoin JM107 dans lesquelles nous avons introduit les deux plasmides compatibles NST-01 et pBBnsy contenant respectivement les gènes de l'α-2,3 sialyltransferase et de la CMP-NeuAc synthase. Cette souche est dépourvue d'activité NanA et est donc incapable de dégrader de l'acide sialique intracellulaire. Par contre elle possède les gènes de la lactose permease (*lacY*) et de la sialyl permease *(nanT)* et est donc capable d'internaliser du lactose et de l'acide sialique exogène. L'acide sialique internalisé peut ainsi être activé en CMP-NeuAc sous l'action de la CMP-NeuAc synthase et transféré sur du lactose intracellulaire sous l'action de l'α-2,3 sialyltransferase.

La souche JM107-nanA-(Nst-01, pBBnsy) et la souche témoin JM107 (Nst-01, pBBnsy) possédant l'activité NanA ont été cultivées à haute densité cellulaire sur glycérol. Du lactose (1,5 g.l⁻¹) de l'IPTG (0,1 mM) et de l'acide sialique (0,6 g.l⁻¹) sont ajoutés au début de la deuxième phase de culture d'une durée de 5 h. Durant toute la durée (17 h) de la troisième phase de la culture, on introduit en continu 100 mg.h⁻¹.L⁻¹ d'acide sialique et 200 mg.h⁻¹.L⁻¹ de lactose.

A la fin de la culture de la souche JM107-nanA-(Nst-01, pBBnsy), le dosage enzymatique du lactose avec et sans traitement par une neuraminidase permet d'estimer la production totale de siallyllactose à 2,6 g.l⁻¹. Cette production est retrouvée en partie dans les cellules bactériennes (1,5 g.l⁻¹ ) et dans le milieu extracellulaire de culture (1,1 g.l⁻¹). Dans le cas de la souche témoin JM107 (Nst-01, pBBnsy), la production de sialyllactose est beaucoup plus faible (150 mg.l⁻¹ ) indiquant que la quasi totalité de l'acide sialique a été dégradée par la bactérie.

Les oligosaccharides intracellulaires et extracellulaires sont purifiés par adsorption sur charbon actif et élution à l'éthanol. Après purification sur résine échangeuse d'anions un seul produit est détecté en HPLC. Le spectre de masse en mode FAB⁺ montre la présence de deux ions quasi-moléculaires [M+H]⁺ à m/z 656 et [M+Na] à m/z ⁺ 678 correspondant au sel de sodium du sialyllactose.

### Exemple 8: Production de dérivés fucosylés du lacto-N-néotétraose

Chez *E. coli* K12, les gènes de biosynthèse du GDP-fucose font partie de l'opéron responsable de la biosynthèse d'un polysaccharide extracellulaire, l'acide colanique (Stevenson et al 1996). L'expression de cet opéron est controlé par un réseau complexe de régulation dans lequel est impliqué la proteine RcsA (Stout et al 1991). La surexpression du gene *rcs*A se traduit ainsi par une surproduction d'acide colanique (Russo et Singh 1993) et par conséquent des gènes de biosynthèse du GDP fucose.

Pour augmenter la disponibilité en GDP-fucose, notre stratégie a consisté à utiliser une souche d'*E. coli* dans laquelle le gène *rcsA* était surexprimé (de manière à surproduire les gène de biosynthèse du GDP-fucose) et dans laquelle un des gènes essentiel à la biosynthèse de l'acide colanique a été inactivé (de manière à supprimer totalement la production d'acide colanique et éviter une compétition pour l'utilisation du GDP-fucose)

Nous avons utilisé la souche JM107-col-DE3 dans laquelle le gène *wcaJ ,* qui est responsable du transfert du premier résidu glucose de l'unité de répétition , a été inactivé selon l'exemple 1 et dans laquelle nous avons introduit soit les deux plasmides pHP0651 et pBBLnt, soit les deux plasmides pHP0651 et pBBLntRcsA. Le plasmide pHP0651 contient le gène *fucT* de l'α -1,3 fucosyltransferase d'*Helicobacter pylori.* Cette fucosyltransférase utilise comme accepteur la N-acetyllactosamine et le lacto-N-néo-tetraose mais pas le lactose (Martin et al 1997). Le plasmide pBBLnt contient les gènes *lgtA* et *lgtB.* Le plasmide pBBLntRcsA contient les gènes *lgtA, lgtB* et *rcsA.*

Les deux souches JM107-col-DE3 (pHP0651, pBBLnt) et JM 107-col-DE3 (pHP0651, pBBLntRcsA) ont été cultivées comme dans l'exemple 3 en présence de 5 g.l⁻¹ de lactose. A la fin de troisième phase de culture la quantité de GlcNAc hydrolysable produite par les deux souches (1,7 g.l⁻¹ ) était comparable à celle obtenu par la souche JM 109 (pCWlgtA,pBBlgtb) dans l'exemple 3. Le dosage colorimétrique du fucose en fin de culture montre une différence importante entre les deux souches avec une production de fucose de lg.l⁻¹ pour la souche JM107 ^{_}col⁻DE3 (pHP0651, pBBLntRcsA) et de seulement 0,25 g.l⁻¹ pour la souche JM107-col-DE3 (pHP0651, pBBLnt). Les oligosaccharides fucosylés sont retrouvés à plus de 70% dans la fraction intracellulaire.

La purification de la fraction intracellulaire par adsorption sur charbon actif et chromatographie d'exclusion stérique sur Biogel P2 permet de séparer quatre composés principaux.

Le composé 1 correspond de par son volume d'élution sur Biogel P2 et sa migration sur couche mince au lacto-*N*-néo-tétraose.

Le spectre de masse du composé 2 montre la présence d'un ion quasi-moléculaire [M+H]⁺ à m/z à 854 correspondant a la masse molaire du lacto-*N*-fucopentaose. La présence d'un ion secondaire à 327 indique que la molécule est fucosylée sur le résidu glucose et possède la structure suivante
β-D-Gal-[1→4]β-D-GlcNAc-[1→3]-β-D-Gal-[1→4]-α-L-Fuc-[1→3])-β-D-Glc

Le spectre de masse du composé 3 majoritaire montre la présence de 3 ions quasi moléculaires à m/z 1000, 1022 et 1038 correspondant aux trois formes [M+H]⁺, [M+Na]⁺ et [M+K]⁺ de la molécule de lacto-*N*-difucohexaose ayant la structure suivante
β-D-Gal-[1→4] β-L-Fuc-[1→3])β-D-GlcNAc-[1→3]-β-D-Gal-[1→4]-(α-L-Fuc-[1→3])-β-D-Glc.

Le spectre de masse du composé 4 permet d'identifier deux ions quasi moléculaires à m/z 1365 et 1388 correspondant aux formes [M+H]⁺et [M+Na]⁺ d'une molécule de lacto-*N*-difucooctaoses. La présence d'un ion secondaire à m/z 512 indique que le residu GlcNAc de l'extrémité non réductrice porte un fucose. Les données de RMN montrent que le proton ¹H d'un résidu fucose est sensible à l'anomérie et que ce residu fucose est donc fixé sur le glucose. Ces résultats permettent de proposer pour le composé 4 la structure suivante :
β-D-Gal-[1→4]]-(α-L-Fuc-[1→3])β-D-GlcNAc-[1→3]β-D-Gal-[1→4]β-D-GlcNAc-[1→3]-β-D-Gal-[1→4]-(α-L-Fuc-[1→3])-β-D-Glc.

### REFERENCES

1. Boons (1996) *Tetrahedron* **52**,1095-1121.
2. Dische Z. Shettles L.B. (1948) *J. Biol. Chem.,* **175**, 160-167.
3. Donnenberg M.S., Kaper J.B.(1991) *Infect. Immun.,* **59** :4310-4317.
4. Geremia R.A., Mergaert P., Geelen D., Van Montagu M., Holsters M. (1994) *Proc. Natl. Acad. Sci. USA* **91,** 2669-2673.
5. Gilbert M., Watson D.C., Cunningham A.M., Jennings M.P., Young N.M., Martin A. , Wakarchuk W.W.(1996) *J. Biol. Chem.,* **271** :28271-28276.
6. Gilbert M, Watson D.C., Warachuk W.W (1997). *Biotechnology Letters,* **19**:417-420.J.
7. Gilbert M., Cunningham A.M., Watson D.C., Martin, A., Richards, J.C., Wakarchuk, W.W. (1997) Eur. J. Biochem. **249**, 187-194.
8. Kamst E., van der Drift K.M.G., Thomas-Oates J.E., Lugtenberg B.J.J., Spaink H.P. (1995) *Escherichia coli. J. Bacteriol.* **177**, 6282-6285.
9. Kovach, M.E., Elzre, P.H., Hill, D.S., Robertson, G.T., Rarris, M.A., Roop II, R.M., Peterson, K.M., (1995). Gene **166,** 175-176.
10. Lee R.T., Lee Y.C. (1974). Carbohyd. Res. **37**, 193-203.
11. Martin, S.L., Edbrooke, M.R., Hodgman, T.C., van den Eijnden, D.H, Bird, M.I., (1997) J. Biol. Chem., **34**, 21349-21356.
12. Mergaert P., D'Haeze W., Geelen D., Promé D. Van Montagu M., Geremia R., Promé J.C., Holsters M. (1995) *J. Biol. Chem.* **270***,* 29217-29223.
13. Plumbridge J.,Vimr E.(1999) *J. Bacteriol.,* **181**:47-54.
14. Reissig, J.L., Strominger, J. L., Leloir, L.F., (1955). J. Biol. Chem. **217**, 959-966.
15. Roy R (1997) Recent developements in the rational design of multivalent glycoconjugates, in Topics curr chem., (eds J.Thiem and H. Driguez), Springer, Heidelberg, pp 241-274.
16. Russo T.A., Singh G. (1993) J. Bacteriol. **175**, 7617-7623.
17. Samain, E., Drouillard, S., Heyraud, A., Driguez, H., Geremia, R.A. (1997). *Carbohydr. Res.* **30**, 235-242.
18. Sambrook, J., Fritsch, E.F., Maniatis, T. (1989) Molecular cloning: a laboratory manual, 2^{nd} ed. Cold Spring Harbor laboratory Press. N.Y.
19. Spaink H.P., Wijfjes A.H.M., van der Drift K.M.G., Haverkamp J., Thomas-Oates J.E., Lugtenberg B.J.J. (1994) *Mol. Microbiol.* **13**, 821-831.
20. Stevenson G., Andrianopoulos K., Hobbs M. , P.R. Reeves P.R. (1996) J. *Bacteriol.,* **178** :4885-4893.
21. Stout V., Torres-Cabassa A., Maurizi M.R., Gutnick D., Gottesman S. (1991) *J. Bacteriol.,* **173** :1738-1747.
22. Yannisch-Perron C., Viera J., Messing J. (1985). Gene, **33**, 103-119.

## Revendications

1. Procédé de production d'un oligosaccharide d'intérêt par une cellule génétiquement modifiée à partir d'au moins un précurseur exogène internalisé sélectionné parmi le lactose, un β-galactoside et l'acide sialique, ledit précurseur intervenant dans la voie de biosynthèse dudit oligosaccharide, ledit procédé comprenant les étapes de :
i) Obtention d'une cellule qui :
• comprend au moins un gène recombinant codant pour un enzyme capable d'effectuer une modification dudit précurseur exogène, nécessaire à la synthèse dudit oligosaccharide à partir dudit précurseur, ainsi que les éléments permettant l'expression dudit gène dans ladite cellule,
• est dépourvue d'activité enzymatique susceptible de dégrader ledit précurseur;
ii) Mise en culture de ladite cellule en présence d'au moins undit précurseur exogène, dans des conditions permettant l'internalisation selon un mécanisme de transport actif dudit précurseur exogène par ladite cellule et la production dudit oligosaccharide par ladite cellule, ladite mise en culture cellulaire étant effectuée sur un substrat carboné dans des conditions permettant l'obtention d'une culture à haute densité cellulaire, comprenant :
a)- une première phase de croissance cellulaire exponentielle assurée par ledit substrat carboné;
b)- une seconde phase de croissance cellulaire limitée par ledit substrat carboné qui est ajouté de manière continue ;
c)- une troisième phase de croissance cellulaire ralentie obtenue en ajoutant de manière continue dans la culture une quantité dudit substrat diminuée par rapport à la quantité de substrat ajoutée à l'étape b) de façon à augmenter la teneur en oligosaccharides produits dans la culture à haute densité cellulaire.

2. Procédé selon la revendication 1 **caractérisé en ce que** ladite cellule comprend en outre au moins un gène codant pour un enzyme capable d'effectuer une modification d'un précurseur endogène intervenant dans la voie de biosynthèse dudit oligosaccharide, ledit enzyme étant identique ou différent de l'enzyme selon la revendication 1, ainsi que les éléments permettant l'expression dudit gène dans ladite cellule et **caractérisé en ce que** ladite cellule est dépourvue d'activité enzymatique susceptible de dégrader ledit précurseur.

3. Procédé selon les revendications 1 et 2 **caractérisé en ce que** ladite cellule est une cellule choisie parmi les bactéries et les levures.

4. Procédé selon la revendication 3 **caractérisé en ce que** la cellule est une bactérie, de préférence de type *Escherichia coli.*

5. Procédé selon l'une des revendications 1 à 4 **caractérisé en ce que** ladite modification est choisie parmi la glycosylation, la sulfatation, l'acétylation, la phosphorylation, la succinylation, la méthylation, l'addition d'un groupement énolpyruvate.

6. Procédé selon l'une des revendications 1 à 5 **caractérisé en ce que** ledit enzyme est un enzyme capable d'effectuer une glycosylation choisi parmi les glycosyl-transférases.

7. Procédé selon la revendication 6 **caractérisé en ce que** ledit enzyme est une glycosyl-transférase choisie parmi la β-1,3-N-acétyl-glucosaminyl-transférase, la β-1,3 galactosyl-transférase, l'α-1,3 N-acétyl-galactosaminyl-transférase, la β-1,3 glucuronosyl-transférase, la β-1,3 N-acétyl-galactosaminyl-transférase, la β-1,4 N-acétyl-galactosaminyl-transférase, la β-1,4-galactosyl-transférase, l'α-1,3-galactosyl transférase, l'α-1,4-galactosyl-transférase, l'α-2,3-sialyl-transférase, l'α-2,6-sialyl-transférase, l'α-2,8-sialyl-transférase, l'α-1,2-fucosyl-transférase, l'α-1,3-fucosyl-transférase, l'α-1,4-fucosyl-transférase.

8. Procédé selon la revendication 1 **caractérisé en ce que** ledit substrat carboné est choisi parmi le glycérol et le glucose.

9. Procédé selon la revendication 1 **caractérisé en ce que** la quantité de substrat ajouté de manière continue dans la culture cellulaire au cours de ladite phase c) est diminuée d'au moins 30%, de préférence 50%, de manière préférée 60% par rapport à la quantité de substrat ajouté de manière continue lors de ladite phase b).

10. Procédé selon l'une des revendications 1 ou 9 **caractérisé en ce que** ledit précurseur est ajouté lors de la phase b).

11. Procédé selon la revendication 1 **caractérisé en ce que** ledit précurseur est le lactose.

12. Procédé selon la revendication 1 **caractérisé en ce que** ledit précurseur est choisi dans le groupe composé :
• de β-galactosides naturels ou synthétiques, de préférence dans le 4-O-β-D-galactopyranosyl-D-fructofuranose (lactulose), le 3-O-β-D-galacto-pyranosyl-D-arabinose, l'allyl-β-D-galactopyranoside ;

13. Procédé selon les revendications 11 ou 12 **caractérisé en ce que** ledit transport actif dudit précurseur est réalisé par la lactose perméase.

14. Procédé selon la revendication 1 **caractérisé en ce que** ledit précurseur est l'acide sialique.

15. Procédé selon la revendication 14 **caractérisé en ce que** ledit transport actif dudit précurseur est réalisé par la perméase Nan T.

16. Procédé selon la revendication 1 **caractérisé en ce que** ledit précurseur est l'acide sialique et le lactose.

17. Procédé selon la revendication 16 **caractérisé en ce que** ledit transport actif dudit précurseur est réalisé par la lactose perméase et la perméase Nan T.

18. Procédé selon la revendication 17 **caractérisé en ce que** ladite cellule a un génotype choisi parmi *LacZ* et/ou *Nan A-.*

19. Procédé selon l'une quelconque des revendications 1 à 18 **caractérisé en ce qu'**il comprend en outre l'addition d'un inducteur dans ledit milieu de culture pour induire l'expression dans ladite cellule dudit enzyme et/ou d'une protéine impliquée dans ledit transport actif.

20. Procédé selon la revendication 19 **caractérisé en ce que** ledit inducteur est l'isopropyl β-D-thiogalactoside (IPTG) et ladite protéine est la lactose perméase.

21. Procédé selon l'une quelconque des revendications 1 à 20. pour la production du trisaccharide 4-O-[3-O-(2-acétamido-2déoxy-β-D-glucopyranosyl)-β-D-galactopyranosyl]-D-glucopyranose, (β-D-GlcNac-[1→3]-β-D-Gal-[1→4]-D-Glc) **caractérisé en ce que** :
• ladite cellule est une bactérie de génotype *LacZ-, LacY*+;
• ledit enzyme est la β-1,3-N-acétyl-glucosaminyl-transfsrase ;
• ledit substrat est le glycérol ;
• ledit inducteur est l'isopropyl β-D-thiogalactoside (IPTG) ;
• ledit précurseur est le lactose.

22. Procédé selon l'une quelconque des revendications 1 à 27, pour la production du lacto-N-néo-tétraose et de polylactosamine (par exemple lacto-N-néo-hexaose, lacta-N-néo-octaose, lacto-N-néo-décaose) **caractérisé en ce que** :
• ladite cellule est une bactérie de génotype *LacZ*-, *LacY*+
• lesdits enzymes sont la β-1,3-N-acétyl-glucasaminyl-transférase et la β- 1,4-galactosyl-transférase
• ledit substrat est le glucose ;
• ledit inducteur est l'isopropyl β-D-thiogalactoside (IPTG) ;
• ledit précurseur est le lactose.

23. Procédé selon la revendication 22, pour la production d'un dérivé sialylé du lacto-N-néotétraose et de polylactosamine (par exemple lacto-N-néo-hexaose, lacto-N-néo-octaose, lacto-N-néo-décaose) **caractérisé en ce qu'**il comprend en outre un dit enzyme choisi parmi l'α-2,3 sialyl-transférase, l'α-2,6 sialyl-transférase, et que ladite cellule a en outre un génotype *NanA-*, *NanT*+ et exprime le gène de la CMP-NeuAc-synthase.

24. Procédé selon la revendication 22 pour la production d'un dérivé fucosylé du lacto-N-néotétraose et de polylactosamine (par exemple lacto-N-néo-hexaose, lacto-N-néo-octaose, lacto-N-néo-décaose) **caractérisé en ce qu'**il comprend en outre un dit enzyme choisi parmi 1'α-1,2 fucosyl-transférase, l'α-1,3 fucosyl-transférase, et que ladite cellule a en outre un génotype Wca J⁻ et surexprime le gène Rcs A.

25. Procédé selon la revendication 21 pour la production d'un dérivé sialylé et fucosylé du lacto-N-neotétraose, lacto-N-néo-décaose **caractérisé en ce qu'**il comprend en outre un dit enzyme choisi parmi le l'α-2,3 sialyl-transférase, l'α-2,6 sialyl-transférase, et en outre un dit enzyme choisi parmi l'α-1,2 fucosyl-transférase, l'α-1,3 fucosyl-transférase, et que ladite cellule a en outre un génotype *NanA-, Nan T*^{*+*}*, Wca J*^{*-*} et surexprime le gène *Rcs A* et le gène de la CMP-NeuAc-synthase.

26. Procédé selon l'une quelconque des revendications 1 à 20, pour la production du 3'-sialyllactose (α-NeuAc-[2→3]-β-D-Gal-[1→4]-β-D-Glc) ou du 6'-sialyllactose (α-NeuAc-[2→6]-β-D-Gal-[1→4]-β-D-Glc) **caractérisé en ce que** :
• ladite cellule est une bactérie de génotype *LacZ-, LacY+ , Nan A*^{*-*}*,* Nan *T*^{*+*};
• lesdits enzymes sont la CMP-NeuAc-synthase et l'α-2,3 sialyl-transférase ou l'α-2,6 sialyl-transférase;
• ledit substrat est le glycérol ;
• ledit inducteur est l'isopropyl-β-D-thiogalactoside (IPTG) ;
• lesdits précurseurs sont le lactose et l'acide sialique.

27. Procédé selon l'une quelconque des revendications 1 à 20 pour la production de 3'fucosyllactose β-D-Gal-[1→4]-(α-L-Fuc-(1→3])-D-Glc ou de 2'fucosyllactose α-L-Fuc-[1→2]-β-D-Gal-[1→4]-D-Glc **caractérisé en ce qu'**il comprend un dit enzyme choisit parmi l'α-1,3 fucosyltransférase ou l'α-1,2 fucosyltransférase, et que la cellule a un génotype *wcaj*^{*-*} *lacZ-* et surexprime le gène *rcsA* et que ledit précurseur est le lactose.

28. Procédé selon l'une quelconque des revendications 1 à 20 pour la production de l'allyl 3-O-(2-acétamido-2déoxy-β-D-glucopyranosyl)-β-D-galactopyranoside (β-D-GlcNac-[1→3]-β-D-Gal-1→O-allyl) **caractérisé en ce que** :
• ladite cellule est une bactérie de génotype *LacZ*-, *LacY+;*
• ledit enzyme est la β-1,3-N-acétyl-glucosaminyl-transférase ;
• ledit substrat est le glycérol ;
• ledit inducteur est l'isopropyl β-D-thiogalactoside (IPTG) ;
• ledit précurseur est l'allyl-β-D-galactopyranoside.

29. Procédé selon l'une quelconque des revendications 1 à 20 pour la production d'analogues du lacto-N-néo-tétraose et de polylactosamines dans lesquels le résidu glucose est remplacé par un groupement allyl **caractérisé en ce que** :
• ladite cellule est une bactérie de génotype LacZ-; *LacY+;*
• lesdits enzymes sont la β-1,3-N-acétyl-glucosaminyl-transférase et la β-1,4-galactosyl-transférase ;
• ledit substrat est le glucose ;
• ledit inducteur est l'isopropyl β-D-thiogalactoside (IPTG) ;
• ledit précurseur est l'allyl-β-D-galactopyranoside.

30. Procédé selon l'une des revendications 23 à 27 pour la production d'analogue d'oligosaccharides dans lesquels le résidu glucose est remplacé par un groupement allyl **caractérisé en ce que** ledit précurseur est l'allyl-β-D galactoside.

31. Procédé selon les revendications 1 à 30 de production d'oligosaccharide marqué par au moins un isotope **caractérisé en ce que** ladite cellule est cultivée sur ledit substrat carboné marqué par ledit isotope et/ou en présence d'un dit précurseur marqué par ledit isotope.

## Patentansprüche

1. Verfahren zur Herstellung eines Oligosaccharids von Interesse durch eine genetisch modifizierte Zelle ausgehend von wenigstens einer internalisierten exogenen Vorstufe, ausgewählt aus Lactose, einem β-Galactosid und Sialinsäure, wobei die Vorstufe an dem Biosyntheseweg des Oligosaccharids beteiligt ist, wobei das Verfahren die folgenden Schritte umfasst:
i) Gewinnung einer Zelle, welche:
• wenigstens ein rekombinantes Gen, welches ein Enzym kodiert, welches in der Lage ist, eine Modifizierung der exogenen Vorstufe, welche für die Synthese des Oligosaccharids ausgehend von der Vorstufe erforderlich ist, auszuführen, wie auch die Elemente, welche die Expression des Gens in der Zelle erlauben, umfasst,
• keine enzymatische Tätigkeit aufweist, welche in der Lage wäre, die Vorstufe abzubauen;
ii) Kultivierung der Zelle in Gegenwart von wenigstens einer solchen exogenen Vorstufe unter Bedingungen, welche die Internalisierung der exogenen Vorstufe durch die Zelle gemäß einem aktiven Transportmechanismus und die Produktion des Oligosaccharids durch die Zelle erlauben, wobei die Zellkultivierung auf einem kohlenstoffhaltigen Substrat ausgeführt wird unter Bedingungen, welche erlauben, eine Kultur mit hoher Zelldichte zu erhalten, umfassend:
a) eine erste Phase einer exponentiellen Zellvermehrung, welche durch das kohlenstoffhaltige Substrat sichergestellt wird;
b) eine zweite Phase einer Zellvermehrung, welche durch das kohlenstoffhaltige Substrat, welches kontinuierlich zugesetzt wird, begrenzt wird;
c) eine dritte Phase einer verlangsamten Zellvermehrung, welche erhalten wird, indem in die Kultur kontinuierlich eine bezogen auf die in Schritt b) zugesetzte Substratmenge verringerte Menge des Substrats hinzugegeben wird derart, dass der Gehalt an in der Kultur mit hoher Zelldichte produzierten Oligosacchariden erhöht wird.

2. Verfahren nach Anspruch 1, **dadurch gekennzeichnet, dass** die Zelle außerdem wenigstens ein Gen, welches ein Enzym kodiert, welches in der Lage ist, eine Modifizierung einer endogenen Vorstufe, welche an dem Biosyntheseweg des Oligosaccharids beteiligt ist, auszuführen, wobei das Enzym identisch mit oder verschieden ist von dem Enzym nach Anspruch 1, wie auch die Elemente, welche die Expression des Gens in der Zelle erlauben, umfasst, und **dadurch gekennzeichnet, dass** die Zelle keine enzymatische Aktivität aufweist, welche in der Lage wäre, die Vorstufe abzubauen.

3. Verfahren nach den Ansprüchen 1 und 2, **dadurch gekennzeichnet, dass** die Zelle eine Zelle, welche unter den Bakterien und den Hefen ausgewählt wird, ist.

4. Verfahren nach Anspruch 3, **dadurch gekennzeichnet, dass** die Zelle ein Bakterium, vorzugsweise vom Typ *Escherichia coli,* ist.

5. Verfahren nach einem der Ansprüche 1 bis 4, **dadurch gekennzeichnet, dass** die Modifizierung unter der Glycosylierung, der Sulfatierung, der Acetylierung, der Phosphorylierung, der Succinylierung, der Methylierung, der Hinzufügung einer Enolpyruvat-Gruppe ausgewählt wird.

6. Verfahren nach einem der Ansprüche 1 bis 5, **dadurch gekennzeichnet, dass** das Enzym ein Enzym, welches in der Lage ist, eine Glycosylierung auszuführen, welches unter den Glycosyltransferasen ausgewählt wird, ist.

7. Verfahren nach Anspruch 6, **dadurch gekennzeichnet, dass** das Enzym eine Glycosyltransferase ist, welche unter der β-1,3-N-Acetylglucosaminyltransferase, der β-1,3-Galactosyltransferase, der α-1,3-N-Acetylgalactosaminyltransferase, der β-1,3-Glucuronosyltransferase, der β-1,3-N-Acetylgalactosaminyltransferase, der β-1,4-N-Acetylgalactosaminyltransferase, der β-1,4-Galactosyltransferase, der α-1,3-Galactosyltransferase, der α-1,4-Galactosyltransferase, der α-2,3-Sialyltransferase, der α-2,6-Sialyltransferase, der α-2,8-Sialyltransferase, der α-1,2-Fucosyltransferase, der α-1,3-Fucosyltransferase, der α-1,4-Fucosyltransferase ausgewählt wird.

8. Verfahren nach Anspruch 1, **dadurch gekennzeichnet, dass** das kohlenstoffhaltige Substrat unter Glycerol und Glucose ausgewählt wird.

9. Verfahren nach Anspruch 1, **dadurch gekennzeichnet, dass** die in die Zellkultur während der Phase c) kontinuierlich hinzugegebene Substratmenge bezogen auf die während der Phase b) kontinuierlich zugesetzte Substratmenge um wenigstens 30%, vorzugsweise 50%, bevorzugt 60% verringert ist.

10. Verfahren nach einem der Ansprüche 1 oder 9, **dadurch gekennzeichnet, dass** die Vorstufe während der Phase b) zugesetzt wird.

11. Verfahren nach Anspruch 1, **dadurch gekennzeichnet, dass** die Vorstufe Lactose ist.

12. Verfahren nach Anspruch 1, **dadurch gekennzeichnet, dass** die Vorstufe ausgewählt wird aus der Gruppe bestehend aus:
• natürlichen oder synthetischen β-Galactosiden, vorzugsweise unter 4-O-β-D-Galactopyranosyl-D-fructofuranose (Lactulose), 3-O-β-D-Galactopyranosyl-D-arabinose, Allyl-β-D-galactopyranosid.

13. Verfahren nach den Ansprüchen 11 oder 12, **dadurch gekennzeichnet, dass** der aktive Transport der Vorstufe durch die Lactosepermease realisiert wird.

14. Verfahren nach Anspruch 1, **dadurch gekennzeichnet, dass** die Vorstufe Sialinsäure ist.

15. Verfahren nach Anspruch 14, **dadurch gekennzeichnet, dass** der aktive Transport der Vorstufe durch die Permease Nan T realisiert wird.

16. Verfahren nach Anspruch 1, **dadurch gekennzeichnet, dass** die Vorstufe Sialinsäure und Lactose ist.

17. Verfahren nach Anspruch 16, **dadurch gekennzeichnet, dass** der aktive Transport der Vorstufe durch die Lactosepermease und die Permease Nan T realisiert wird.

18. Verfahren nach Anspruch 17, **dadurch gekennzeichnet, dass** die Zelle einen unter *LacZ-* und/oder *Nan A*- ausgewählten Genotyp hat.

19. Verfahren nach einem der Ansprüche 1 bis 18, **dadurch gekennzeichnet, dass** es außerdem die Zugabe eines Induktors in das Kulturmedium, um die Expression des Enzyms und/oder eines an dem aktiven Transport beteiligten Proteins in der Zelle zu induzieren, umfasst.

20. Verfahren nach Anspruch 19, **dadurch gekennzeichnet, dass** der Induktor Isopropyl-β-D-thiogalactosid (IPTG) ist und das Protein die Lactosepermease ist.

21. Verfahren nach einem der Ansprüche 1 bis 20 für die Herstellung des Trisaccharids 4-O-[3-O-(2-Acetamido-2-desoxy-β-D-glucopyranosyl)- β-D-galactopyranosyl]-D-glucopyranose, (β-D-GlcNac-[1→3]-β-D-Gal-[1→4]-D-Glc), **dadurch gekennzeichnet, dass**:
• die Zelle ein Bakterium von *LacZ-,LacY+*-Genotyp ist;
• das Enzym die β-1,3-N-Acetylgtucosaminyltransferase ist;
• das Substrat Glycerol ist;
• der Induktor Isopropyl-β-D-thiogalactosid (IPTG) ist;
• die Vorstufe Lactose ist.

22. Verfahren nach einem der Ansprüche 1 bis 21 für die Herstellung von Lacto-N-neo-tetraose und von Polylactosamin (beispielsweise Lacto-N-neo-hexaose, Lacto-N-neo-octaose, Lacto-N-neo-decaose), **dadurch gekennzeichnet, dass**:
• die Zelle ein Bakterium von *LacZ-,LacY*+-Genotyp ist;
• die Enzyme die β-1,3-N-Acetylglucosaminyltransferase und die β-1,4-Galactosyltransferase sind;
• das Substrat Glucose ist;
• der Induktor Isopropyl-β-D-thiogalactosid (IPTG) ist;
• die Vorstufe Lactose ist.

23. Verfahren nach Anspruch 22 für die Herstellung eines sialylierten Derivats von Lacto-N-neo-tetraose und von Polylactosamin (beispielsweise Lacto-N-neo-hexaose, Lacto-N-neo-octaose, Lacto-N-neo-decaose), **dadurch gekennzeichnet, dass** es außerdem ein solches Enzym, ausgewählt unter der α-2,3-Sialyltransferase, der α-2,6-Sialyltransferase, umfasst und dass die Zelle außerdem einen NanA-,NanT+-Genotyp aufweist und das Gen der CMP-NeuAc-Synthase exprimiert.

24. Verfahren nach Anspruch 22 für die Herstellung eines fucosylierten Derivats von Lacto-N-neo-tetraose und von Polylactosamin (beispielsweise Lacto-N-neo-hexaose, Lacto-N-neo-octaose, Lacto-N-neo-decaose), **dadurch gekennzeichnet, dass** es außerdem ein solches Enzym, ausgewählt unter der α-1,2-Fucosyltransferase, der α-1,3-Fucosyltransferase, umfasst und dass die Zelle außerdem einen *Wca J*⁻-Genotyp aufweist und das Gen Rcs A überexprimiert.

25. Verfahren nach Anspruch 22 für die Herstellung eines sialylierten und fucosylierten Derivats von Lacto-N-neo-hexaose, Lacto-N-neo-decaose, **dadurch gekennzeichnet, dass** es außerdem ein solches Enzym, ausgewählt unter der α-2,3-Sialyltransferase, der α-2,6-Sialyltransferase, und außerdem ein solches Enzym, ausgewählt unter der α-1,2-Fucosyltransferase, der α-1,3-Fucosyltransferase, umfasst und dass die Zelle außerdem einen *NanA-,NanT+, Wca J*^{*-*}-Genotyp aufweist und das Gen *Rcs A* und das Gen der CMP-NeuAc-Synthase überexprimiert.

26. Verfahren nach einem der Ansprüche 1 bis 20 für die Herstellung von 3-Sialyllactose (α-NeuAc-[2→3]-β-D-Gal-[1→4]-β-D-Glc) oder 6-Sialyllactose (α-NeuAc-[2→6]-β-D-Gal-[1→4]-β-D-Glc), **dadurch gekennzeichnet, dass**:
• die Zelle ein Bakterium von *LacZ- ,LacY+-,NanA*^{*-*}*,NanT*^{*+*}*-Genotyp* ist;
• die Enzyme die CMP-NeuAc-Synthase und die α-2,3-Sialyltransferase oder die α-2,6-Sialyltransferase sind;
• das Substrat Glycerol ist;
• der Induktor Isopropyl-β-D-thiogalactosid (IPTG) ist;
• die Vorstufen Lactose und Sialinsäure sind.

27. Verfahren nach einem der Ansprüche 1 bis 20 für die Herstellung von 3-Fucosyllactose (β-D-Gal-[1→4]-(α-L-Fuc-[1→3])-D-Glc) oder 2-Fucosyllactose (α-L-Fuc-[1→2]-β-D-Gal-[1→4]-D-Glc), **dadurch gekennzeichnet, dass** es ein solches Enzym, ausgewählt unter der α-1,3-Fucosyltransferase oder der α-1,2-Fucosyltransferase, umfasst und dass die Zelle einen *wcaj lacZ-*-Genotyp aufweist und das Gen *rcsA* überexprimiert und dass die Vorstufe Lactose ist.

28. Verfahren nach einem der Ansprüche 1 bis 20 für die Herstellung von Allyl-3-O-(2-acetamido-2-desoxy-β-D-glucopyranosyl)-β-D-galactopyranosid (β-D-GlcNac-[1→3]-β-D-Gal-1→O-allyl), **dadurch gekennzeichnet, dass**:
• die Zelle ein Bakterium von *LacZ-,LacY+*-Genotyp ist;
• das Enzym die β-1,3-N-Acetylglucosaminyltransferase ist;
• das Substrat Glycerol ist;
• der Induktor Isopropyl-β-D-thiogalactosid (IPTG) ist;
• die Vorstufe Allyl-β-D-galactopyranosid ist.

29. Verfahren nach einem der Ansprüche 1 bis 20 für die Herstellung von Analoga von Lacto-N-neo-tetraose und von Polylactosaminen, in welchen der Glucoserest durch eine Allylgruppe ersetzt ist, **dadurch gekennzeichnet, dass**:
• die Zelle ein Bakterium von *LacZ-,LacY*+-Genotyp ist;
• die Enzyme die β-1,3-N-Acetylglucosaminyltransferase und die β-1,4-Galactosyltransferase sind;
• das Substrat Glucose ist;
• der Induktor Isopropyl-β-D-thiogalactosid (IPTG) ist;
• die Vorstufe Allyl-**β**-D-galactopyranosid ist.

30. Verfahren nach einem der Ansprüche 23 bis 27 für die Herstellung von Analoga von Oligosacchariden, in welchen der Glucoserest durch eine Allylgruppe ersetzt ist, **dadurch gekennzeichnet, dass** die Vorstufe Allyl-β-D-galactosid ist.

31. Verfahren nach den Ansprüchen 1 bis 30 zur Herstellung eines Oligosaccharids, welches durch wenigstens ein Isotop markiert ist, **dadurch gekennzeichnet, dass** die Zelle auf dem kohlenstoffhaltigen Substrat, welches durch das Isotop markiert ist, und/oder in Gegenwart einer durch das Isotop markierten Vorstufe kultiviert wird.

## Claims

1. Method for producing an oligosaccharide of interest by a genetically modified cell starting with at least.one internalized exogenous precursor selected from lactose, a β-galactoside and sialic acid, said precursor being involved in the biosynthetic pathway of said oligosaccharide, said method comprising the steps of:
(i) obtaining a cell that
• comprises at least one recombinant gene encoding an enzyme capable of modifying said exogenous precursor necessary for the synthesis of said oligosaccharide from said precursor, and also the components for expressing said gene in said cell,
• lacks any enzymatic activity liable to degrade said precursor;
(ii) culturing said cell in the presence of at least one said exogenous precursor, under conditions enabling the internalization according to a mechanism of active transport of said exogenous precursor by said cell and the production of said oligosaccharide by said cell, said cell culturing being carried out on a carbon-based substrate under conditions allowing the production of a culture with a high cell density, comprising:
a)- a first phase of exponential cell growth ensured by said carbon-based substrate,
b)- a second phase of cell growth limited by said carbon-based substrate which is added continuously,
c)- a third phase of slowed cell growth obtained by continuously adding to the culture an amount of said substrate that is less than the amount of substrate added in step b) so as to increase the content of oligosaccharides produced in the high cell density culture.

2. Method according to Claim 1, **characterized in that** said cell also comprises at least one gene encoding an enzyme capable of modifying an endogenous precursor involved in the biosynthetic pathway of said oligosaccharide, said enzyme being identical to or different than the enzyme used in the method described above, and also to the components for expressing said gene in said cell and **characterized in that** said cell lacks any enzymatic activity liable to degrade said precursor.

3. Method according to Claim 1 or 2, **characterized in that** said cell is a cell chosen from bacteria and yeasts.

4. Method according to Claim 3, **characterized in that** the cell is a bacterium, preferably of *Escherichia coli* type.

5. Method according to one of Claims 1 to 4, **characterized in that** said modification is chosen from glycosylation, sulphatation, acetylation, phosphorylation, succinylation, methylation, and addition of an enolpyruvate group.

6. Method according to one of Claims 1 to 5, **characterized in that** said enzyme is an enzyme capable of performing a glycosylation, chosen from glycosyl-transferases.

7. Method according to Claim 6, **characterized in that** said enzyme is a glycosyl-transferase chosen from β-1,3-N-acetyl-glucosaminyl-transferase, β-1,3-galactosyl-transferase, α-1,3-N-acetyl-galactosaminyl-transferase, β-1,3-glucuronosyl-transferase, β-1,3-N-acetyl-galactosaminyl-transferase, β-1,4-N-acetyl-galactosaminyl-transferase, β-1,4-galactosyl-transferase, α-1,3-galactosyl-transferase, α-1,4-galactosyl-transferase, α-2,3-sialyl-transferase, α-2,6-sialyl-transferase, α-2,8-sialyl-transferase, α-1,2-fucosyl-transferase, α-1,3-fucosyl-transferase and α-1,4-fucosyl-transferase.

8. Method according to Claim 1, **characterized in that** said carbon-based substrate is chosen from glycerol and glucose.

9. Method according to Claim 1, **characterized in that** the amount of substrate added continuously to the cell culture during said phase c) is at least 30% less, preferentially 50% and preferably 60% less than the amount of substrate added continuously during said phase b).

10. Method according to either of Claims 1 and 9, **characterized in that** said precursor is added during phase b).

11. Method according to Claim 1, **characterized in that** said precursor is lactose.

12. Method according to Claim 1, **characterized in that** said precursor is chosen from the group composed
• of natural or synthetic β-galactosides, preferably from 4-O-β-D-galactopyranosyl-D-fructofuranose (lactulose), 3-O-β-D-galactopyranosyl-D-arabinose and allyl-β-D-galactopyranoside.

13. Method according to either of Claims 11 and 12, **characterized in that** said active transport of said precursor is performed by lactose permease.

14. Method according to Claim 1, **characterized in that** said precursor is sialic acid.

15. Method according to Claim 14, **characterized in that** said active transport of said precursor is performed by NanT permease.

16. Method according to Claim 1, **characterized in that** said precursor is sialic acid and lactose.

17. Method according to Claim 16, **characterized in that** said active transport of said precursor is performed by lactose permease and NanT permease.

18. Method according to Claim 17, **characterized in that** said cell has a genotype chosen from *LacZ*^{*-*} and/or *NanA*^{*-*}*.*

19. Method according to any one of Claims 1 to 18, **characterized in that** it also comprises the addition of an inducer to said culture medium to induce the expression in said cell of said enzyme and/or of a protein involved in said active transport.

20. Method according to Claim 19, **characterized in that** said inducer is isopropyl β-D-thiogalactoside (IPTG) and said protein is lactose permease.

21. Method according to any one of Claims 1 to 20, for the production of the trisaccharide 4-O-[3-O-(2-acetamido-2-deoxy-β-D-glucopyranosyl)-β-D-galactopyranosyl]-D-glucopyranose, (β-D-GlcNac-[1→3]-β-D-Gal-[1→4]-D-Glc), **characterized in that**:
• said cell is a bacterium of *LacZ*^{*-*}*, LacY*⁺ genotype;
• said enzyme is β-1,3-N-acetyl-glucosaminyl-transferase;
• said substrate is glycerol;
• said inducer is isopropyl β-D-thiogalactoside (IPTG);
• said precursor is lactose.

22. Method according to any one of Claims 1 to 21, for the production of lacto-N-neo-tetraose and polylactosamine (for example, lacto-N-neo-hexaose, lacto-N-neo-octaose, lacto-N-neo-decaose), **characterized in that**:
• said cell is a bacterium of *LacZ*^{*-*}*, LacY*⁺ genotype;
• said enzymes are β-1,3-N-acetyl-glucosaminyl-transferase and β-1,4-galactosyl-transferase;
• said substrate is glucose;
• said inducer is isopropyl-β-D-thiogalactoside (IPTG) ;
• said precursor is lactose.

23. Method according to Claim 22, for the production of a sialyl derivative of lacto-N-neo-tetraose and of polylactosamine (for example, lacto-N-neo-hexaose, lacto-N-neo-octaose, lacto-N-neo-decaose), **characterized in that** it also comprises a said enzyme chosen from α-2,3-sialyl-transferase and α-2,6-sialyl-transferase, and **in that** said cell also has a *NanA*^{*-*}*, NanT*^{*+*} genotype and expresses the gene for CMP-NeuAc-synthase.

24. Method according to Claim 22, for the production of a fucosyl derivative of lacto-N-neo-tetraose and of polylactosamine (for example, lacto-N-neo-hexaose, lacto-N-neo-octaose, lacto-N-neo-decaose), **characterized in that** it also comprises a said enzyme chosen from α-1,2-fucosyl-transferase and α-1,3-fucosyl-transferase, and **in that** said cell also has a WcaJ⁻ genotype and overexpresses the RcsA gene.

25. Method according to Claim 22, for the production of a sialyl and fucosyl derivative of lacto-N-neo-tetraose, lacto-N-neo-decaose, **characterized in that** it also comprises a said enzyme chosen from α-2,3-sialyl-transferase and α-2,6-sialyltransferase, and also a said enzyme chosen from α-1,2-fucosyl-transferase and α-1,3-fucosyl-transferase, and **in that** said cell also has a *NanA*^{*-*}*, NanT*^{*+*}*, WcaJ*^{*-*} genotype and overexpresses the *RcsA* gene and the gene for CMP-NeuAc-synthase.

26. Method according to any one of Claims 1 to 20, for the production of 3'-sialyllactose (α-NeuAc-[2→3]-β-D-Gal- [1→4]-β-D-Glc) or 6'-sialyllactose (α-NeuAc-[2→6]-β-D-Gal-[1→4]-β-D-Glc), **characterized in that**:
• said cell is a bacterium of *LacZ*^{*-*}*, LacY*^{*+*}*, NanA*^{*-*} or *NanT*⁺ genotype;
• said enzymes are CMP-NeuAc-synthase and α-2,3-sialyl-transferase or α-2,6-sialyl-transferase;
• said substrate is glycerol;
• said inducer is isopropyl-β-D-thiogalactoside (IPTG);
• said precursors are lactose and sialic acid.

27. Method according to any one of Claims 1 to 20, for the production of 3'-fucosyllactose (β-D-Gal-[1→4]-(α-L-Fuc-[1→3]-D-Glc) or 2' -fucosyllactose, α-L-Fuc-[1→2]-β-D-Gal-[1→4]-D-Glc **characterized in that** it comprises a said enzyme chosen from α-1,3-fucosyl-transferase or α-1,2-fucosyl-transferase, and **in that** the cell has a *wcaj*^{*-*} *lacZ*^{*-*} genotype and overexpresses the *rcsA* gene and **in that** said precursor is lactose.

28. Method according to any one of Claims 1 to 20, for the production of allyl 3-O-(2-acetamido-2-deoxy-β-D-glucopyranosyl)-β-D-galactopyranoside, (β-D-GlcNac-[1→3]-β-D-Gal-1→O-allyl) , **characterized in that**:
• said cell is a bacterium of *LacZ*^{*-*}*, LacY'* genotype;
• said enzyme is β-1,3-N-acetyl-glucosaminyl-transferase;
• said substrate is glycerol;
• said inducer is isopropyl β-D-thiogalactoside (IPTG);
• said precursor is allyl-β-D-galactopyranoside.

29. Method according to any one of Claims 1 to 20, for the production of analogues of lacto-N-neo-tetraose and of polylactosamines in which the glucose residue is replaced with an allyl group, **characterized in that** .
• said cell is a bacterium of *LacZ*^{*-*}*, ,LacY*⁺ genotype;
• said enzymes are β-1,3-N-acetyl-glucosaminyl-transferase and β-1,4-galactosyl-transferase;
• said substrate is glucose;
• said inducer is isopropyl β-D-thiogalactoside (IPTG);
• said precursor is allyl-β-D-galactopyranoside.

30. Method according to one of Claims 23 to 27, for the production of oligosaccharide analogues in which the glucose residue is replaced with an allyl group, **characterized in that** said precursor is allyl-β-D-galactoside.

31. Method according to Claims 1 to 30, for producing an oligosaccharide labelled with at least one isotope, **characterized in that** said cell is cultured on said carbon-based substrate labelled with said isotope and/or in the presence of a said precursor labelled with said isotope.
